(19) Europäisches Patentamt
European Patent Office
Office européen des brevets

(11) EP 1 189 890 B1

(12) # FASCICULE DE BREVET EUROPEEN

(45) Date de publication et mention de la délivrance du brevet:
**19.04.2006 Bulletin 2006/16**

(21) Numéro de dépôt: **00945864.7**

(22) Date de dépôt: **04.07.2000**

(51) Int Cl.:
***C07D 243/04*** *(2006.01)* ***A61K 31/55*** *(2006.01)*
***C07D 487/04*** *(2006.01)* ***C07D 513/04*** *(2006.01)*
***C07D 401/12*** *(2006.01)* ***C07D 405/12*** *(2006.01)*
***C07D 413/12*** *(2006.01)* ***C07D 513/14*** *(2006.01)*
***C07D 409/12*** *(2006.01)* *C07D 487/04 (2006.01)*
*C07D 243/00 (2006.01)* *C07D 235/00 (2006.01)*
*C07D 513/04 (2006.01)* *C07D 277/00 (2006.01)*
*C07D 243/00 (2006.01)*

(86) Numéro de dépôt international:
**PCT/EP2000/006230**

(87) Numéro de publication internationale:
**WO 2001/002373 (11.01.2001 Gazette 2001/02)**

(54) **DIHYDROBENZODIAZEPINES ET LEUR UTILISATION DANS LE TRAITEMENT DE DYSLIPIDEMIES**

DIHYDROBENZODIAZEPINEN UND DEREN VERWENDUNG ZUR BEHANDLUNG VON DYSLIPIDÄMIEN

DIHYDROBENZODIAZEPINS AND THEIR USE FOR TREATING DYSLIPIDEMIA

(84) Etats contractants désignés:
**AT BE CH CY DE DK ES FI FR GB GR IE IT LI LU MC NL PT SE**

(30) Priorité: **06.07.1999 FR 9908714**

(43) Date de publication de la demande:
**27.03.2002 Bulletin 2002/13**

(73) Titulaire: **Merck Patent GmbH**
**64293 Darmstadt (DE)**

(72) Inventeurs:
- **BERTHELON, Jean-Jacques**
  **F-69005 Lyon (FR)**
- **GUERRIER, Daniel**
  **F-69230 Saint Genis Laval (FR)**
- **BRUNET, Michel**
  **F-69780 Toussieu (FR)**
- **ZEILLER, Jean-Jacques**
  **F-69008 Lyon (FR)**
- **CONTARD, Francis**
  **F-69003 Lyon (FR)**
- **AUSSEIL, Fréderic,**
  **Le Musset Bâtiment C - App. 26**
  **F-31500 Toulouse (FR)**

(56) Documents cités:
**WO-A-96/05188** **FR-A- 2 540 871**
**US-A- 3 780 023**

- **LINDA L. SETESCAK ET AL.: "4-Aryl-4,5-dihydro-3H-1,3-benzodiazepines .3. 2-Phenyl and 2-amino analogues as potential antihypertensive agents" JOURNAL OF MEDICINAL CHEMISTRY., vol. 27, no. 3, - 1984 pages 401-404, XP002134185 AMERICAN CHEMICAL SOCIETY. WASHINGTON., US ISSN: 0022-2623**
- **TIMOTHY JEN ET AL.: "Amidines. 4. Synthesis of tricyclic guanidines related to 1,2,3,5-tetrahydroimidazo(2,1-b)quinazolin e, a new antihypertensive agent" JOURNAL OF MEDICINAL CHEMISTRY., vol. 16, no. 4, - 1973 pages 407-411, XP002134186 AMERICAN CHEMICAL SOCIETY. WASHINGTON., US ISSN: 0022-2623**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne des dihydrobenzodiazépines utilisables dans le traitement de dyslipidémies, de l'athérosclérose, du diabète et de ses complications.

La maladie cardiovasculaire demeure dans la plupart des pays une des principales maladies et la principale cause de mortalité. Environ un tiers des hommes développent une maladie cardiovasculaire majeure avant l'âge de 60 ans, les femmes présentent un risque inférieur (rapport 1 à 10). Avec la vieillesse (après 65 ans, les femmes deviennent aussi vulnérables aux maladies cardiovasculaires que les hommes), cette maladie prend encore de l'ampleur. Les maladies vasculaires comme la maladie coronaire, l'accident vasculaire cérébral, la resténose et la maladie vasculaire périphérique demeurent la première cause de mortalité et de handicap à travers le monde.

Tandis que le régime alimentaire et le style de vie peuvent accélérer le développement de maladies cardiovasculaires, une prédisposition génétique conduisant à des dyslipidémies est un facteur significatif dans les accidents cardiovasculaires et les décès. Le développement de l'athérosclérose semble être relié principalement à la dyslipidémie, ce qui signifie des niveaux anormaux de lipoprotéines dans le plasma sanguin. Cette dysfonction est particulièrement évidente dans la maladie coronaire, le diabète et l'obésité.

Le concept destiné à expliquer le développement de l'athérosclérose a été principalement orienté sur le métabolisme du cholestérol et sur le métabolisme des triglycérides.

Chez l'homme, l'hypertriglycéridémie est une atteinte relativement commune avec 10% des hommes de 35 à 39 ans présentant des concentrations plasmatiques supérieures à 250 mg/dl (LaRosa J.C., L.E. Chambless, M.H. Criqui, I.D. Frantz, C.J. Glueck, G. Heiss, and J.A. Morisson, 1986. Circulation <u>73</u> : Suppl. 1.12-29.). Chez certains individus, la perturbation est d'origine génétique mais pour d'autres des causes secondaires, telles que la consommation excessive d'alcool, l'obésité, le diabète ou l'hypothyroïdisme, prédominent.

Les causes génétiques clairement identifiées de l'hypertriglycéridémie sont l'homozygotie pour des allèles dysfonctionnels de la LPL ou de l'apo CII [Fojo S.S., J.L. de Gennes, U. Beisiegel, G. Baggio, S.F. Stahlenhoef, J.D. Brunzell, and H.B. Brewer, Jr 1991. Adv. Exp. Med. Biol. <u>285 :</u> 329-333 ; Brunzell, J.D. 1995. In the Metabolic Basis of Inherited Disease, 6th ed. C. Scriver, A. Sly and D. Valle, editors. Mc Graw-Hill, Inc., New York. 1913-1932.]. Ces conditions ne surviennent cependant que dans un cas sur un million et sont considérées comme rares. Il existe des preuves, provenant d'études réalisées chez l'homme et chez des souris déficientes en LPL [Brunzell, J.D. 1995. In the Metabolic Basis of Inherited Disease, 6th ed. C. Scriver, A. Sly and D. Valle, editors. Mc Graw-Hill, Inc., New York. 1913-1932 ; Coleman T., et al. 1995. J. Biol. Chem. <u>270</u> : 12518-12525 ; Aalto-Setälä K., Weinstock P.H., Bisgaier C.L., Lin Wu, Smith J.D. and Breslow J.L., 1996. Journal of Lipid Research, <u>37,</u> 1802-1811] montrant que l'hétérozygotie pour un allèle dysfonctionnel de la LPL peut contribuer à l'hypertriglycéridémie avec toutefois une fréquence d'occurrence faible dans la population. La concentration plasmatique d'apolipoprotéine CIII (apo CIII), régulée par l'expression du gène apo CIII, associée ou non à une cause secondaire, peut être une nouvelle et plus fréquente cause de l'hypertriglycéridémie chez l'Homme [Weinstock P.H., C.L. Bisgaier, K. Aalto-Setälä, H. Radner, R. Ramakrishnan, S. Levak-Frank, A.D. Essenburg, R. Zechner, and J.L. Breslow, 1995. J.Clin. Invest. <u>96 :</u> 2555-2568].

L'apo CIII est un composant des lipoprotéines très basse densité (very low density lipoproteins ou VLDL), des chylomicrons et des lipoprotéines haute densité (high density lipoproteins ou HDL).

De nombreuses études montrent que l'apo CIII joue un rôle important dans le métabolisme des lipoprotéines riches en triglycérides (TGRL). Des études cliniques montrent une forte corrélation entre l'apo CIII plasmatique et la concentration en triglycérides [Schonfeld. G., P.K. George, J. Miller, P. Reilly, and J. Witztum, 1979. <u>28 :</u> 1001-1010. ; Shoulders C.C., et al. 1991. Atherosclerosis <u>87 :</u> 239-247 ; Le N-A., J.C. Gibson, and H.N. Ginsberg, 1988. J. Lipid Res. <u>29 :</u> 669-677]. De plus, des études épidémiologiques montrent une association entre certains allèles de l'apo CIII et la concentration en triglycérides [Rees A., J. Stocks, C.R. Sharpe, M.A. Vella. C.C. Shoulders, J. Katz, N.I. Jowett, F.E. Baralle, and D.J. Galton, 1985 J. Clin. Invest. <u>76 :</u> 1090-1095 ; Aalto-Setälä, et al. 1987. Atherosclerosis <u>66 :</u> 145-152 ; Tas, S. 1989. Clin. Chem. <u>35 :</u> 256-259 ; Ordovas J.M. , et al. 1991. Atherosclerosis 87 : 75-86 ; Ahn, Y.I., et al. 1991. Hum. Hered <u>41 :</u> 281-289 : Zeng Q., M. Dammerman, Y. Takada, A. Matsunage, J.I. Breslow and J. Sasaki, 1994. Hum. Genet <u>95</u> : 371-375].

L'apo CIII a la capacité d'inhiber l'activité de la lipoprotéine lipase (LPL) [C.S. Wang, W.J. Mc Connathy, H.U. Kloer and P. Alaupovic, J. Clin. Invest., <u>75,</u> 384 (1984)] et de diminuer l'élimination des "remnants" des lipoprotéines riches en triglycérides (TGRL) par la voie des récepteurs de l'apolipoprotéine E [F. Shelburne, J. Hanks, W. Meyers and S. Quarfordt, J. Clin. Invest., <u>65,</u> 652 (1980) ; E. Windler and R.J. Havel, J. Lipid Res., <u>26.</u> 556, (1985)]. Chez les patients déficients en apo CIII, le catabolisme des TGRL est accéléré [H. N. Ginsberg, N.A. Le, I.A. Goldberg, J.C. Gibson, A. Rubinstein, P. Wang-Iverson, R. Norum and W.V. Brown, J. Clin. Invest., <u>78,</u> 1287 (1986)]. A l'inverse, la sur-expression de l'apo CIII humaine chez des souris transgéniques est associée à une hypertriglycéridémie sévère [Y. Ito, N. Azrolan, A. O'Connell, A. Walsh and J.L. Breslow, Science, 249, 790 (1990)].

Par ces mécanismes, l'apo CIII entraine la réduction du catabolisme des TGRL conduisant à une hausse de la concentration en triglycérides. La réduction de la concentration plasmatique d'apo CIII apparaît donc d'un intérêt

certain lorsque la baisse de la triglycéridémie est recherchée comme objectif thérapeutique chez des populations à risque.

Les composés de l'invention sont des dihydrobenzodiazépines capables de diminuer la sécrétion d'apo CIII.

Les composés de l'invention ont pour formule I:

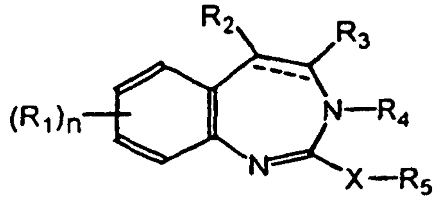

I

dans laquelle

les pointillés indiquent la présence éventuelle d'une double liaison ;

$R_1$ représente $(C_1-C_{18})$alkyle éventuellement halogéné, $(C_1-C_{18})$alcoxy éventuellement halogéné, halogène, nitro, hydroxy ou $(C_6-C_{18})$aryle (éventuellement substitué par $(C_1-C_{10})$alkyle éventuellement halogéné, $(C_1-C_{12})$alcoxy éventuellement halogéné, halogène, nitro ou hydroxy) ;

n représente 0, 1, 2, 3 ou 4 ;

$R_2$ et $R_3$ représentent indépendamment l'un de l'autre hydrogène ; $(C_1-C_{18})$alkyle éventuellement halogéné ; $(C_1-C_{18})$ alcoxy ; $(C_6-C_{18})$aryle ; $(C_6-C_{18})$aryl-$(C_1-C_{12})$alkyle ; hétéroaryle ; hétéroaryl$(C_1-C_{12})$alkyle ; $(C_6-C_{18})$aryloxy ; $(C_6-C_{18})$ aryl-$(C_1-C_{12})$alcoxy ; hétéroaryloxy ; ou hétéroaryl$(C_1-C_{12})$alcoxy ; dans lesquels les parties aryle et hétéroaryle de ces radicaux sont éventuellement substituées par halogène, $(C_1-C_{12})$alcoxy éventuellement halogéné, $(C_1-C_{12})$alkyle éventuellement halogéné, nitro et hydroxy ;

X représente S, O ou -NT où T représente un atome d'hydrogène, $(C_1-C_{12})$alkyle, $(C_6-C_{18})$aryle, $(C_6-C_{18})$aryl-$(C_1-C_{12})$ alkyle ou $(C_6-C_{18})$arylcarbonyle ;

$R_4$ et $R_5$ forment ensemble le groupe $-CR_6=CR_7-$ dans lequel $CR_6$ est relié à X et dans lequel :

$R_6$ représente un atome d'hydrogène ; $(C_1-C_{18})$alkyle ; $(C_3-C_{12})$cycloalkyle ; $(C_6-C_{18})$aryle ; carboxy-$(C_1-C_{12})$ alkyle ; $(C_1-C_{12})$alcoxy-carbonyl-$(C_1-C_{12})$alkyle ; hétéroaryle ; $(C_6-C_{18})$aryl-$(C_1-C_{12})$alkyle ; et hétéroaryl-$(C_1-C_{12})$ alkyle ; dans lesquels les parties aryle et hétéroaryle de ces radicaux sont éventuellement substituées par $(C_1-C_{12})$ alkyle, $(C_1-C_{12})$alcoxy, hydroxy, nitro, halogène ou di$(C_1-C_{12})$atcoxyphosphoryl$(C_1-C_{12})$alkyle ;

$R_7$ représente un atome d'hydrogène ; hydroxy ; di$(C_1-C_{12})$alkylamino$(C_1-C_{12})$alkyle ; $(C_1-C_{18})$alkyle éventuellement halogéné ; carboxy ; carboxy$(C_1-C_{12})$alkyle éventuellement substitué par amino ; $(C_1-C_{12})$alcoxycarbonyle ; $(C_6-C_{18})$aryle ; hétéroaryle ; $(C_6-C_{18})$aryl-$(C_1-C_{12})$alkyle ; ou hétéroaryl-$(C_1-C_{12})$alkyle ; $(C_6-C_{18})$aryle condensé à un hétérocycle insaturé, éventuellement substitué sur la partie hétérocycle par oxo ; $(C_3-C_{12})$cycloalkyle ;

dans lesquels les parties aryle, hétérocycle, cycloalkyle et hétéroaryle de ces radicaux sont éventuellement substituées par halogène ; hydroxy ; hydroxy-$(C_1-C_{12})$alcoxy ; $(C_1-C_{12})$alkyle éventuellement halogéné ; $(C_1-C_{12})$alcoxy éventuellement halogéné ; carboxy ; $(C_1-C_{12})$alcoxycarbonyle ; nitro ; cyano ; cyano$(C_1-C_{18})$alkyle ; $(C_1-C_{18})$alkylcarbonyloxy ; $(C_2-C_{12})$alkylène ; $(C_1-C_{12})$alkylènedioxy ; $(C_1-C_{12})$alkylthio ; $(C_6-C_{18})$arylthio éventuellement substitué par un ou plusieurs substituants Su tels que définis ci-dessus ; di$(C_1-C_{12})$alkylamino ; un groupe de formule:

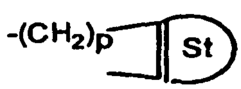

dans lequel p = 0, 1, 2, 3 ou 4 et où St représente $(C_6-C_{18})$aryle ; $-alk-Cy-NH-SO_2-Ar$ où alk représente $(C_1-C_{12})$alkyle, Cy représente $(C_3-C_{12})$cycloalkyle éventuellement substitué par un ou plusieurs substituants Su tels que définis ci-dessous et Ar représente $(C_6-C_{18})$aryle éventuellement substitué par un ou plusieurs substituants Su tels que définis ci-dessous ; $-Cy-alk-NH-SO_2-Ar$ où Cy, alk et Ar sont tels que définis ci-dessus ; -alk-Cy où alk et Cy sont tels que définis ci-dessus ; -alk-Cy-alk'-NH-CO-alk" où alk et Cy sont tels que définis ci-dessus et alk', alk" représentent indépendamment $(C_1-C_{12})$alkyle ; di$(C_1-C_{12})$alcoxyphosphoryl$(C_1-C_{12})$alkyle ; $(C_6-C_{18})$aryle éventuellement substitué par un ou plusieurs substituants Su tels que définis ci-dessous ; $(C_6-C_{18})$aryloxy éventuellement substitué par un ou plusieurs substituants Su tels que définis ci-dessous ; $(C_6-C_{18})$arylcarbonyle éventuellement substitué par un ou plusieurs substituants Su tels

que définis ci-dessous ; ($C_6$-$C_{18}$)arylsulfonyle éventuellement substitué par un ou plusieurs substituants Su tels que définis ci-dessous ; ($C_6$-$C_{18}$)aryl-($C_1$-$C_{12}$)alcoxy dont la partie aryle est éventuellement substituée par un ou plusieurs substituants Su tels que définis ci-dessous ; hétérocycle saturé éventuellement substitué par un ou plusieurs substituants Su tels que définis ci-dessous ; ($C_6$-$C_{18}$)aryl-($C_1$-$C_{12}$)alkyle éventuellement substitué par un ou plusieurs substituants Su tels que définis ci-dessous ;

Su est choisi parmi hydroxy, halogène, cyano, nitro, ($C_1$-$C_{12}$)alkyle éventuellement halogéné et ($C_1$-$C_{12}$)alcoxy éventuellement halogéné ;

ou bien $R_6$ et $R_7$ forment ensemble une chaîne alkylène en $C_3$-$C_{12}$ éventuellement interrompue par un atome d'azote lequel est éventuellement substitué par ($C_1$-$C_{12}$)alkyle ou ($C_6$-$C_{18}$)aryle ou ($C_6$-$C_{18}$)aryl-($C_1$-$C_{12}$)alkyle, le cycle formé par $CR_6$=$CR_7$ étant éventuellement condensé à ($C_6$-$C_{18}$)aryle (les parties aryle de ces radicaux étant éventuellement substituées par halogène, nitro, hydroxy, ($C_1$-$C_{12}$)alkyle éventuellement halogéné ou ($C_1$-$C_{12}$)alcoxy éventuellement halogéné) ;

et leurs sels pharmaceutiquement acceptables avec des acides ou des bases, étant entendu que les composés suivants sont exclus du cadre de l'invention:

(a) X = S ; n = 0 ; $R_2$ représente méthyle et $R_3$ représente un atome d'hydrogène : $R_4$ et $R_5$ forment ensemble le groupe $-CR_6=CR_7-$ dans lequel $CR_6$ est relié à X, $R_6$ et $R_7$ forment ensemble une chaîne $-(CH_2)_3-$ ou $-(CH_2)_4-$ ou bien $R_6$ représente un atome d'hydrogène ou un groupe propyle et $R_7$ est un groupe phényle éventuellement substitué par $-OCH_3$ ou un groupe hydroxy.

Il doit être entendu que les composés de formule I dans laquelle X = S ; n = 0 ; $R_2$ représente méthyle et $R_3$ représente un atome d'hydrogène ; $R_4$ et $R_5$ forment ensemble le groupe $-CR_6=CR_7-$ dans lequel $CR_6$ est relié à X, $R_6$ et $R_7$ forment ensemble une chaîne $-(CH_2)_3-$ ou $-(CH_2)_4-$ ou bien $R_6$ représente un atome d'hydrogène ou un groupe propyle et $R_7$ est un groupe phényle éventuellement substitué par $-OCH_3$ ou un groupe hydroxy, sont exclus du cadre de l'invention.

Les sels pharmaceutiquement acceptables avec des acides ou des bases des composés de formule I font également partie de l'invention.

J. Heterocycl. Chem. 1969, 6 (4), 491 décrit des dérivés de benzodiazépine présentant une structure analogue à celle du tétramisole (chlorhydrate de DL-2,3,5,6-tétrahydro-6-phénylimidazo[2,1-b]thiazole) lequel est un puissant agent anthelmintique. Parmi ces composés, ceux dont la structure répond à la formule I ci-dessus ont été exclus, par disclaimer du cadre de l'invention.

L'invention vise, non seulement les composés de formule I, mais également leurs sels.

Lorsque le composé de formule I comprend une fonction acide, et par exemple une fonction carboxylique, celui-ci peut former un sel avec une base minérale ou organique.

A titre d'exemple de sels avec des bases organiques ou minérales, on peut citer les sels formés avec des métaux et notamment des métaux alcalins, alcalino-terreux et de transition (tels que le sodium, le potassium, le calcium, le magnésium, l'aluminium), ou avec des bases comme l'ammoniac ou des amines secondaires ou tertiaires (telles que la diéthylamine, la triéthylamine, la pipéridine, la pipérazine, la morpholine) ou avec des acides aminés basiques, ou avec des osamines (telles que la méglumine) ou avec des amino alcools (tels que le 3-amino-butanol et le 2-aminoéthanol).

Lorsque le composé de formule I comprend une fonction basique, et par exemple un atome d'azote, celui-ci peut former un sel avec un acide organique ou minéral.

Les sels avec des acides organiques ou minéraux sont par exemple les chlorhydrate, bromhydrate, sulfate, hydrogénosulfate, dihydrogénophosphate, citrate, maléate, fumarate, 2-naphtalènesulfonate et paratoluène sulfonate.

L'invention couvre également les sels permettant une séparation ou une cristallisation convenable des composés de formule I tels que l'acide picrique, l'acide oxalique ou un acide optiquement actif, par exemple l'acide tartrique, l'acide dibenzoyltartrique, l'acide mandélique ou l'acide camphosulfonique.

La formule I englobe tous les types d'isomères géométriques et de stéroisomères des composés de formule I.

Selon l'invention, le terme "alkyle" désigne un radical hydrocarboné linéaire ou ramifié comportant de préférence de 1 à 8 atomes de carbone, mieux encore de 1 à 12 atomes de carbone, par exemple de 1 à 10 et notamment de 1 à 6. Des exemples en sont notamment les groupes méthyle, éthyle, propyle, isopropyle, butyle, tert-butyle, isobutyle, pentyle, hexyle, heptyle, octyle, nonyle, décyle, undécyle, dodécyle, tridécyle, tétradécyle, pentadécyle, hexadécyle, heptadécyle ou octadécyle.

Le terme "alcoxy" désigne un groupe alkyle tel que défini ci-dessus lié à un atome d'oxygène. Des exemples en sont les radicaux méthoxy, éthoxy, isopropyloxy, butoxy et hexyloxy.

Par "éventuellement halogéné", on entend éventuellement substitué par un ou plusieurs atomes d'halogène.

Lorsque le groupe alkyle est éventuellement halogéné, on préfère qu'il représente perfluoroalkyle et notamment pentafluoroéthyle ou trifluorométhyle.

Lorsque le groupe alcoxy est halogéné, on préfère qu'il représente $-O-CHF_2$ ou qu'il soit perfluoré. Des exemples de radicaux perfluorés sont $-OCF_3$ et $-O-CF_2-CF_3$.

Par groupe alkylène, on entend des groupes alkylène linéaires ou ramifiés, c'est-à-dire des radicaux bivalents qui sont des chaînes alkyle bivalentes linéaires ou ramifiées.

Le terme "cycloalkyle" désigne des groupements hydrocarbonés, saturés qui peuvent être mono- ou polycycliques et comprennent préférablement de 3 à 18 atomes de carbone, mieux encore de 3 à 12 atomes de carbone, par exemple de 3 à 8.

Les groupes cycloalkyle polycycliques sont constitués de monocycles condensés deux à deux (par exemple orthocondensés ou péricondensés), c'est-à-dire présentant au moins deux à deux, deux atomes de carbone en commun.

On préfère plus particulièrement les groupements cycloalkyle monocycliques tels que cyclopropyle, cyclobutyle, cyclopentyle, cyclohexyle, cycloheptyle, cyclooctyle, cyclononyle, cyclodécyle, cycloundécyle et cyclododécyle.

Parmi les cycloalkyle polycycliques, on peut citer adamantyle, norbomyle ou le groupe de formule :

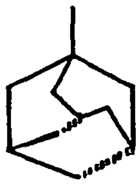

Par "cycloalcényle", on entend selon l'invention un groupe cycloalkyle tel que défini ci-dessus, présentant une ou plusieurs doubles liaisons, de préférence une double liaison.

Par "halogène" on entend un atome de fluor, de chlore, de brome ou d'iode.

Par "alcényle", on entend une chaine hydrocarbonée linéaire ou ramifiée comprenant une ou plusieurs doubles liaisons. Des exemples de groupes alcényle particulièrement préférés sont les groupes alcényle portant une seule double liaison tels que $-CH_2-CH_2-CH=C(CH_3)_2$, vinyle ou allyle.

Le terme "aryle" représente un groupement hydrocarboné mono- ou polyclique aromatique comprenant préférablement de 6 à 18 atomes de carbone, par exemple de 6 à 14 atomes de carbone, notamment de 6 à 10 atomes de carbone.

Chaque groupe aryle polycyclique comprend deux ou plusieurs noyaux aromatiques monocycliques, condensés deux à deux, c'est-à-dire présentant deux à deux au moins deux atomes de carbone en commun.

Des exemples préférés de groupes aromatiques polycycliques sont les groupes bicycliques, tricycliques et tétracycliques.

Parmi ceux-ci, on peut mentionner les groupes phényle, naphtyle, anthryle, phénanthryle, pyrényle, chrysényle et naphtacényle.

Le terme hétéroaryle désigne un radical mono- ou polycyclique aromatique comprenant un ou plusieurs hétéroatomes choisis parmi O, N, S ou P. Préférablement, l'hétéroaryle comprend 1 à 3 hétéroatomes choisis parmi O, N et S.

Lorsque le radical est un radical aromatique polycyclique, celui-ci est constitué de deux ou plusieurs noyaux monocycliques aromatiques condensés deux à deux, chaque noyau monocyclique comportant ou non un ou plusieurs hétéroatomes endocycliques.

De préférence, le radical hétéroaryle polycyclique est bicyclique ou tricyclique.

Avantageusement, le groupe hétéroaryle monocyclique et les noyaux monocycliques formant l'hétéroaryle polycyclique sont constitués de 5 à 7 chaînons. Des exemples d'hétéroaryle monocycliques sont les groupes furyle, thiényle, pyrrolyle, oxazolyle, isoxazolyle, thiazolyle, isothiazolyle, imidazolyle, pyrazolyle, oxadiazolyle, triazolyle, thiadiazolyle, pyridyle, pyridazinyle, pyrazinyle et triazinyle.

Des exemples d'hétéroaryle polycycliques sont indolizine, indole, isoindole, benzofurane, benzothiophène, indazole, benzimidazole, benzothiazole, purine, quinolizine, quinoline, isoquinoline, cinnoline, phtalazine, quinazoline, quinoxaline, naphthyridine, ptéridine, pyrazolotriazine, thiazolopyrimidine, pyrazolopyrimidine, carbazole, acridine, phénazine, phénothiazine, phénoxazine ou le groupe de formule :

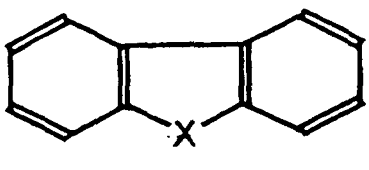

où X est O ou S.

Un exemple d'hétéroaryle est ($C_6$-$C_{18}$)aryle condensé à un hétérocycle aromatique tel qu'un hétérocycle aromatique de 5 à 7 chaînons comprenant un, deux ou trois hétéroatomes endocycliques choisis parmi O, N et S.

Par hétérocycle saturé ou insaturé, on entend un groupe mono- ou polycyclique comprenant un ou plusieurs hétéroatomes choisis parmi O, N, S et P. On préfère que l'hétérocycle comprenne un à trois hétéroatomes choisis parmi O, N et S. Lorsque l'hétérocycle est polycyclique, il comprend deux ou plusieurs noyaux monocycliques saturés ou insaturés comportant préférablement de 5 à 7 chaînons, condensés deux à deux.

Lorsque l'hétérocycle est monocyclique, il comporte 5 à 7 chaînons.

Parmi les hétérocycles polycycliques, on préfère les hétérocycliques bicycliques ou tricycliques.

Des exemples d'hétérocycles saturés sont le tétrahydrofurane, le tétrahydrothiophène, le tétrahydropyrrole, le tétrahydrooxazole, le dioxolane, le tétrahydrothiazole, le tétrahydroimidazole, le tétrahydropyrazole, le tétrahydroisoxazole, le tétrahydroisothiazole, le tétrahydrooxadiazole, le tétrahydrotriazole, le tétrahydrothiadiazole, la pipéridine, le dioxane, la morpholine, le dithiane, la thiomorpholine, la pipérazine, le trithiane.

On peut également citer parmi les hétérocycles saturés, les dérivés saturés des hétéroaryles polycycliques énumérés ci-dessus à titre de radicaux préférés.

Des exemples d'hétérocycles insaturés sont les dérivés insaturés des hétérocycles saturés mentionnés ci-dessus ainsi que les dérivés insaturés des hétéroaryles mentionnés ci-dessus.

Par hétérocycle insaturé, on entend un hétérocycle non aromatique comprenant une ou plusieurs insaturations de type éthylénique.

De préférence l'hétérocycle insaturé comprend une seule double liaison. Des exemples préférés d'hétérocycles insaturés sont dihydrofuryle, dihydrothiényle, dihydropyrrolyle, pyrrolinyle, oxazolinyle, thiazolinyle, imidazolinyle, pyrazolinyle, isoxazolinyle, isothiazolinyle, oxadiazolinyle, pyranyle et les dérivés mono- insaturés de la pipéridine, du dioxane, de la pipérazine, du trithiane, de la morpholine, du dithiane, de la thiomorpholine, ainsi que tétrahydropyridazinyle, tétrahydropyrimidinyle, et tétrahydrotriazinyle.

Lorsque Z ou $R_7$ comprend ou représente ($C_6$-$C_{10}$) aryle éventuellement condensé à un hétérocycle insaturé éventuellement substitué par oxo, l'hétérocyle insaturé présente préférablement au moins une seule insaturation en commun avec le groupe aryle.

Des exemples de tels groupes aryle condensé à un hétérocycle insaturé sont notamment :

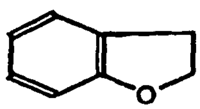

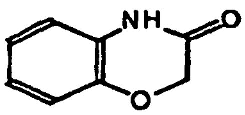

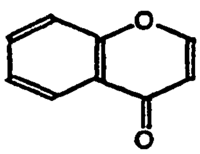

Lorsque Z ou $R_7$ comprend un groupe de formule:

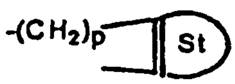

on préfère que p représente 0 ou 1 et St représente phényle.

Préférablement, les extrémités 1 et 2 de ce radical sont rattachées sur deux atomes de carbone adjacents de ladite partie aryle, hétérocycle, cycloalkyle ou hétéroaryle. De manière préférée, St représente phényle. A titre d'exemple on peut mentionner le radical Z de formule :

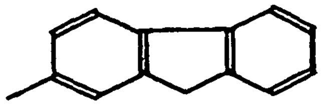

et le radical $R_7$ de formule :

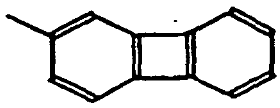

de l'exemple 119 ci-après.

Selon l'invention, l'expression "éventuellement substitué par" signifie généralement "éventuellement substitué par un ou plusieurs des radicaux cités".

A titre d'exemple, lorsque $R_1$ représente ($C_6$-$C_{10}$) aryle, le groupe aryle est éventuellement substitué par un ou plusieurs radicaux choisis parmi :

- ($C_1$-$C_6$) alkyle éventuellement halogéné ;
- ($C_1$-$C_6$) alcoxy ;
- halogène;
- nitro ; et
- hydroxy.

Néanmoins, le nombre de substituants est limité par le nombre possible de substitutions.

Ainsi, lorsque $R_6$ et $R_7$ forment ensemble une chaîne alkylène interrompue par un atome d'azote, celui-ci ne peut-être substitué que par un seul radical choisi parmi alkyle, aryle et arylalkyle.

Un premier groupe des composés de l'invention est constitué des dérivés tricycliques dans lesquels $R_4$ et $R_5$ forment ensemble le groupe $-CR_6=CR_7-$, étant entendu que $R_6$ et $R_7$ ne forment pas ensemble une chaîne alkylène éventuellement interrompue par un atome d'azote.

Un second groupe des composés de l'invention est constitué des dérivés tétracycliques dans lesquels $R_4$ et $R_5$ forment ensemble le groupe $-CR_6=CR_7-$ où $R_6$ et $R_7$ forment ensemble une chaîne alkylène éventuellement interrompue par un atome d'azote.

Lorsque $R_6$ et $R_7$ forment ensemble une chaîne alkylène éventuellement interrompue par un atome d'azote, le cycle formé par $CR_6=CR_7$ peut être condensé à un groupe ($C_6$-$C_{18}$)aryle éventuellement substitué par un ou plusieurs groupes Su.

De manière préférée, $CR_6=CR_7$ forme le groupe :

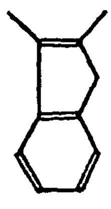

Selon l'invention, un premier groupe de composés préférés (groupe 1) est constitué des composés de formule I dans laquelle X représente -NT où T est tel que défini ci-dessus et $R_4$ et $R_5$ forment ensemble $-CR_6 = CR_7-$

Parmi ces composés on préfère ceux dans lesquels $R_6$ représente un atome d'hydrogène ; et $R_7$ représente hydroxyle ; ou ($C_6$-$C_{10}$) aryle éventuellement substitué par halogène, nitro, hydroxy, ($C_1$-$C_6$) alkyle éventuellement halogéné ou ($C_1$-$C_6$) alcoxy.

Tout particulièrement $R_7$ est choisi parmi hydroxy et phényle.

Des significations préférées de T sont un atome d'hydrogène et ($C_1$-$C_6$) alkyle, par exemple méthyle.

Un second groupe de composés préférés (groupe 2) est constitué des composés de formule I dans laquelle X représente S;

$R_4$ et $R_5$ forment ensemble le groupe $-CR_6=CR_7-$ dans lequel

$R_6$ représente un atome d'hydrogène, ($C_1$-$C_6$)alkyle, ($C_6$-$C_{10}$)aryle (éventuellement substitué par halogène, hydroxy, nitro, ($C_1$-$C_6$)alkyle ou ($C_1$-$C_6$)alcoxy), carboxy-($C_1$-$C_6$)alkyle, ou bien ($C_1$-$C_6$)alcoxycarbonyl-($C_1$-$C_6$)alkyle ; et $R_7$ représente un atome d'hydrogène : hydroxy ; di($C_1$-$C_6$)alkylamino($C_1$-$C_6$)alkyle ; ($C_1$-$C_{10}$)alkyle ; ($C_1$-$C_6$) alcoxycarbonyle ; ($C_6$-$C_{10}$)aryle ; hétéroaryle ; ($C_6$-$C_{10}$)aryl-($C_1$-$C_6$)alkyle ; les parties aryle et hétéroaryle de ces radicaux étant éventuellement substituées par ($C_1$-$C_6$)alcoxycarbonyle, halogène, hydroxy, ($C_1$-$C_6$)alkyle, ($C_6$-$C_{10}$)aryle, (ce dernier étant éventuellement substitué par halogène, ($C_1$-$C_6$)alkyle éventuellement halogéné, ($C_1$-$C_6$)alcoxy ou nitro) ou ($C_6$-$C_{10}$)aryle condensé à un hétérocycle aromatique ou insaturé de 5 à 7 chaînons comprenant un, deux ou trois hétéroatomes endocycliques choisis parmi O, N et S) ; ou bien $R_6$ et $R_7$ forment ensemble une chaîne alkylène interrompue par un atome d'azote éventuellement substitué par ($C_6$-$C_{10}$)aryl-($C_1$-$C_6$)alkyle dans lequel la partie aryle est éventuellement substituée par halogène, ($C_1$-$C_6$)alkyle éventuellement halogéné, ($C_1$-$C_6$)alcoxy, hydroxy ou nitro.

Parmi ces composés on préfère notamment ceux dans lesquels l'un ou plusieurs des substituants $R_4$, $R_5$, $R_6$ et $R_7$ sont définis comme suit :

- $R_4$ et $R_5$ forment ensemble -$CR_6$ = $CR_7$ - dans lequel l'un de $R_6$ ou $R_7$, ou les deux sont tels que définis ci-dessous en (i), (ii) ou (iii) :

  (i) % représente un atome d'hydrogène ; ($C_1$-$C_6$) alkyle ; phényle éventuellement substitué par halogène, ($C_1$-$C_6$) alkyle, ($C_1$-$C_6$)alcoxy, hydroxy ou nitro ; carboxy -($C_1$-$C_6$) alkyle ; ou ($C_1$-$C_6$) alcoxycarbonyl -($C_1$-$C_6$) alkyle ;
  (ii) $R_7$ représente un atome d'hydrogène ; hydroxy ; di ($C_1$-$C_6$) alkylamino ($C_1$-$C_6$) alkyle ; ($C_1$-$C_{10}$) alkyle ; ($C_1$-$C_6$) alcoxycarbonyle ; naphtyle ; phényle éventuellement substitué par halogène, ($C_1$-$C_6$) alcoxycarbonyle, hydroxy, phényle (lui-même éventuellement substitué par halogène, hydroxy, ($C_1$-$C_6$) alkyle éventuellement halogéné, ($C_1$-$C_6$) alcoxy, ($C_1$-$C_6$) alcoxycarbonyle ou nitro) ou phényle condensé à dihydrofuryle, dihydrothiényle ou dihydropyrrolyle ; pyridyle ; furyle ; thiényle ; pyrrolyle ; ou benzyle ;
  (iii) $R_6$ et $R_7$ forment ensemble une chaîne alkylène interrompue par un atome d'azote éventuellement substitué par phényl -($C_1$-$C_6$) alkyle dans lequel la partie alkyle est éventuellement substituée par halogène.

Parmi les composés préférés des groupes 1 et 2, on préfère que l'un au moins de n, $R_1$, $R_2$ et $R_3$ soit tel que défini ci-dessous :

- $R_3$ représente un atome d'hydrogène :

- $R_2$ représente un atome d'hydrogène ou un groupe ($C_6$-$C_{10}$) aryle éventuellement substitué par halogène. ($C_1$-$C_6$) alcoxy, ($C_1$-$C_6$) alkyle éventuellement halogéné, nitro ou hydroxy ;
- $R_1$ représente un atome d'halogène ;
- n représente 0, 1 ou 2, mieux encore n représente 0 ou 1. Plus préférablement n est 0.

Les composés des exemples 1 à 67 ci-après sont préférés.
Parmi ces composés, on préfère tout particulièrement les :

3-(biphényl-4-yl)-5,6-dihydrothiazolo[2,3-b]-1,3-benzodiazépine (exemple 4);
3-(2-furyl)-5,6-dihydrothiazolo[2,3-b]-1,3-benzadiazépine (exemple 43);
3-[4-(éthoxy-carbonyl)phényl]-5,6-dihydrothiazolo-[2,3-b]-1,3-benzodiazépine (exemple 36):
3-(biphényl-3-yl)-5.6-dihydrothiazolo[2,3-b]-1,3-benzodiazépine (exemple 38);
3-(3,4-dihydroxyphényl)-5,6-dihydrothiazolo[2,3-b]-1,3-benzodiazépine (exemple 59) ; et
3-(biphényl-4-yl)-7-chloro-5,6-dihydrothiazolo[2,3-b]-1,3-benzodiazépine (exemple 66).

Les composés de formule I peuvent être préparés simplement en utilisant l'un des procédés ci-dessous.

**B) Cas des composés de formule 1 dans laquelle X représente S, $R_4$ et $R_5$ forment ensemble le groupe -$CR_6$=$CR_7$- et les pointillés ne représentent rien.**

Ces composés peuvent être préparés selon l'invention, par réaction d'une $\alpha$-halogénocétone de formule IVb:

$R_7$-CO-CH$R_6$-$Hal^3$ IVb

dans laquelle $R_6$ et $R_7$ sont tels que définis ci-dessus et $Hal^3$ représente un atome d'halogène, avec une thione de formule IIa :

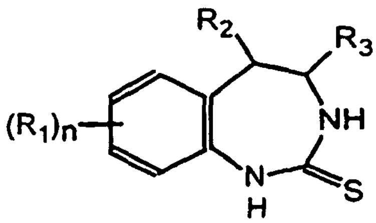

IIa

dans laquelle $R_1$, n, $R_2$ et $R_3$ sont tels que définis ci-dessus pour la formule I, dans un acide carboxylique aliphatique en $C_2$-$C_6$ en tant que solvant, à une température comprise entre 90 et 130° C.

Les conditions exactes de mise en oeuvre seront déterminées par l'homme du métier en fonction de la réactivité des composés en présence.

Comme exemple d'acide carboxylique, on peut citer l'acide acétique, l'acide propionique, l'acide butyrique, l'acide pivalique, et l'acide valérique.

Il est possible, dans le cadre de l'invention, d'opérer en présence d'un mélange de solvants incluant un ou plusieurs acides carboxyliques aliphatiques et éventuellement un ou plusieurs autres solvants polaires, miscibles, inertes vis-à-vis des composés en présence.

De tels solvants additionnels sont par exemple des alcools aliphatiques monohydroxylés en $C_2$-$C_6$ tels que l'éthanol, l'isopropanol et le tert-butanol.

Une plage préférée de température va de 100 à 125° C.

Il peut être commode d'opérer au reflux du solvant, et notamment lorsque le solvant utilisé est l'acide acétique.

**C) Cas des composés de formule I dans laquelle les pointillés ne représentent rien, X représente NH, $R_4$ et $R_5$ forment ensemble -$CR_6$=$CR_7$- et $R_7$ n'est pas le groupe hydroxy.**

Selon l'invention, ces composés peuvent être préparés simplement en deux étapes par mise en oeuvre du procédé suivant.

Dans une première étape, on fait réagir un sulfure de formule V :

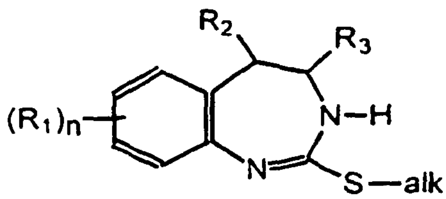

V

dans laquelle $R_1$, n, $R_2$ et $R_3$ sont tels que définis pour I ci-dessus et alk représente ($C_1$-$C_6$)alkyle avec un dérivé protégé de l'acétone de formule VI :

$$NH_2\text{-}CHR_6\text{-}CO\text{-}R_7 \qquad VI$$

dans lequel le groupe carbonyle de $R_6$ est protégé par un groupe protecteur labile en milieu acide, $R_6$ et $R_7$ étant tels que définis ci-dessus.

Des exemples de groupes protecteurs de la fonction carbonyle, labiles en milieu acide, sont donnés dans "Protective Groups in Organic Synthesis, Greene T.W. et Wuts P.G.M., ed. John Wiley et Sons, 1991 et dans Protecting Groups, Kocienski P.J., 1994, Georg Thieme Verlag.

De façon particulièrement avantageuse, le groupe carbonyle peut être protégé sous la forme de cétal, cyclique ou non cyclique.

Ainsi, le dérivé protégé de la cétone de formule VI réagissant avec le sulfure V a préférablement pour formule la formule VIa suivante :

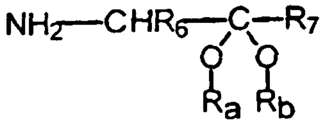
VIa

dans laquelle $R_6$ et $R_7$ sont tels que définis ci-dessus pour I et $R_a$, $R_b$ sont indépendamment $(C_1-C_6)$alkyle ou bien forment ensemble une chaîne $(C_2-C_6)$alkylène linéaire ou ramifiée, de préférence une chaîne $(C_2-C_3)$alkylène.

Les cétals préférés sont notamment les 1,3-dioxolanes et les cétals méthyliques.

On peut néanmoins envisager de protéger le groupement carbonyle par d'autres groupes protecteurs tels que dithio- et hémithiocétals ou par formation d'éther d'énol, d'éther de thioénol, de thiazolidines ou d'imidazolidines.

Le solvant utilisé pour cette réaction est un solvant polaire capable de dissoudre les réactifs en présence. A titre de solvant, on peut ainsi sélectionner un nitrile tel que l'acétonitrile ou l'isobutyronitrile.

Lorsque la réaction est mise en oeuvre à partir du cétal VIa, on obtient, à l'issue de la première étape, le composé de formule :

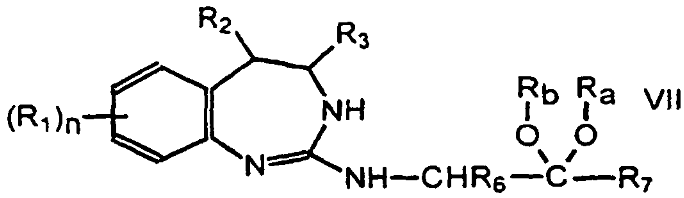
VII

dans laquelle n, $R_1$, $R_2$, $R_3$, $R_6$, $R_7$, $R_a$ et $R_b$ sont tels que définis ci-dessus pour les formules 1 et Via. Le composé résultant de la réaction de II sur le dérivé protégé de la cétone de formule VI, et par exemple le composé VII ci-dessus, est alors traité en milieu acide de façon à provoquer la cyclisation.

En ce but on peut utiliser indifféremment un acide de Brönsted ou un acide de Lewis, un acide minéral ou un acide organique.

Des exemples d'acides appropriés sont notamment l'acide acétique, l'acide formique, l'acide oxalique, l'acide méthanesulfonique, l'acide p-toluènesulfonique, l'acide trifluoroacétique, l'acide trifluorométhanesulfonique, des acides de Lewis tels que le trichlorure de bore, le trifluorure de bore, le tribromure de bore, ou bien encore l'acide chlorhydrique.

La réaction est généralement effectuée entre 15 et 50° C, notamment entre 20 et 30° C.

Le solvant utilisé pour la réaction dépend de l'acide mis en oeuvre. Lorsque l'acide est l'acide chlorhydrique, la réaction est avantageusement conduite dans un $(C_1-C_6)$alcanol tel que l'éthanol.

Le procédé ci-dessus conduit à la préparation de composés de formule I dans lesquels T représente un atome d'hydrogène.

De façon à synthétiser le composé correspondant de formule I dans lequel T représente $(C_1-C_6)$alkyle, $(C_6-C_{10})$ aryle ou $(C_6-C_{10})$aryl- $(C_1-C_6)$alkyle, on fait réagir le composé I obtenu pour lequel T représente hydrogène avec un réactif halogéné de formule Hal-T où T représente $(C_1-C_6)$alkyle, $(C_6-C_{10})$aryle ou $(C_6-C_{10})$aryl- $(C_1-C_6)$alkyle et Hal représente un atome d'halogène, en présence d'une base appropriée.

Des exemples de base sont notamment les bases organiques telles que la N-méthylmorpholine, la triéthylamine, la tributylamine, la diisopropyléthylamine, la dicyclohexylamine, la N-méthylpipéridine, la pyridine, la 4-(1-pyrrolidinyl) pyridine, la picoline, la 4-(N,N-diméthylamino)pyridine, la N,N-diméthylaniline et la N,N-diéthylaniline.

Les conditions de mise en oeuvre de cette réaction sont connues de l'homme du métier.

**D) Cas des composés de formule I dans laquelle les pointillés ne représentent rien, X représente -NT où T est distinct d'un atome d'hydrogène, $R_4$ et $R_5$ forment ensemble le groupe $-CR_6=CR_7-$ et $R_7$ représente hydroxy.**

Ces composés peuvent être préparés par réaction d'un sulfure de formule V :

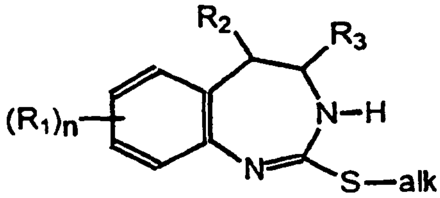

V

dans laquelle n, $R_1$, $R_2$, $R_3$, $R_4$ et alk sont tels que définis ci-dessus, avec un dérivé de formule VIII :

$HTN-CHR_6-CO-Y$ VIII

dans laquelle T et $R_6$ sont tels que définis ci-dessus pour la formule I et Y est un groupe partant, à une température comprise entre 50 et 150° C, de préférence à une température comprise entre 60 et 100° C.

A titre de groupe partant, on peut mentionner un atome d'halogène, un groupe $(C_1-C_6)$alcoxy, un groupe imidazolyle ou un groupe $(C_6-C_{10})$aryl-$(C_1-C_6)$alcoxy.

Cette réaction est généralement conduite dans un solvant polaire et notamment un nitrile tel que l'acétonitrile ou l'isobutyronitrile. On préfère utiliser l'acétonitrile comme solvant.

**F) Cas des composés de formule I dans laquelle les pointillés ne représentent rien, X = S, $R_4$ et $R_5$ forment ensemble $-CR_6=CR_7-$ et $R_7$ représente hydroxy.**

Ces composés sont facilement préparés par réaction d'une thione de formule IIa :

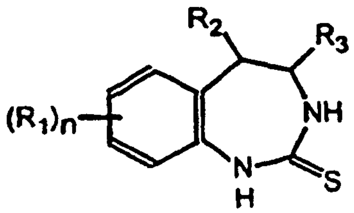

IIa

dans laquelle n, $R_1$, $R_2$ et $R_3$ sont tels que définis ci-dessus pour la formule I. avec un dérivé halogéné de formule X :

$Hal^4-CHR_6-CO-Y$ X

dans laquelle $Hal^4$ représente halogène et $R_6$ et Y sont tels que définis ci-dessus pour la formule VIII.

Cette réaction est de préférence réalisée dans un hydrocarbure aromatique en $C_6-C_{10}$ de type toluène ou benzène. La température à laquelle est réalisée la réaction est généralement comprise entre 80 et 130° C, par exemple entre 100 et 120° C. Des conditions préférées sont par exemple le reflux du toluène.

**G) Cas des composés de formule I dans laquelle les pointillés ne représentent rien, X = S, $R_4$ et $R_5$ forment ensemble -CH=CH-.**

Selon l'invention, ces composés sont préparés en faisant réagir sur la thione suivante de formule XI :

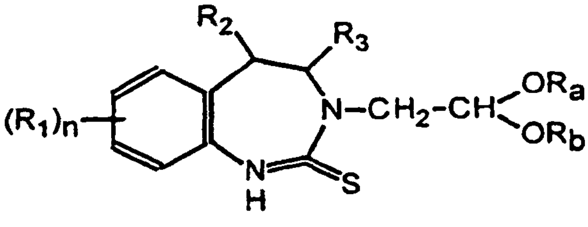

XI

dans laquelle n, $R_1$. $R_2$, $R_3$, $R_a$ et $R_b$ sont tels que définis ci-dessus pour les formules I et VII, un acide fort tel que l'acide sulfurique ou l'acide chlorhydrique, ou bien encore l'un des acides énumérés ci-dessus, dans le cas de la variante C.

Selon ce procédé, la température réactionnelle nécessaire dépend de la force de l'acide utilisé.

Généralement, une température comprise entre 10 et 40° C suffit, par exemple entre 20 et 30° C.

Cette réaction peut être conduite en milieu aqueux. Dans ce cas, le milieu réactionnel obtenu doit être homogène.

**H) Cas des composés de formule I dans laquelle les pointillés ne représentent rien, X représente O, $R_4$ et $R_5$ forment ensemble $-CR_6=CR_7-$**

Ces composés sont préparés par cyclisation thermique d'un composé de formule XII :

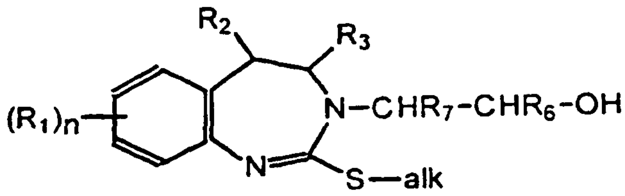

XII

dans laquelle n, $R_1$, $R_2$, $R_3$, $R_6$ et $R_7$ sont tels que définis ci-dessus pour la formule I et alk représente $(C_1-C_6)$alkyle, puis déshydrogénation du composé résultant de formule XIII :

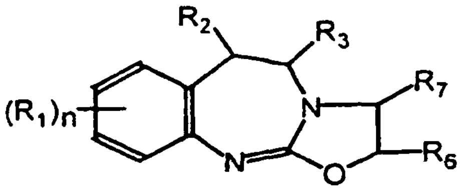

XIII

selon les procédés classiques de la chimie organique, de façon à obtenir le composé attendu de formule 1. La cyclisation thermique peut être par exemple réalisée dans un alcool aliphatique monohydroxylé en $C_2-C_6$ tel que l'éthanol, l'isopropanol ou le tert-butanol comme solvant à une température comprise entre 80 et 160° C.

**I) Cas des composés de formule 1 dans laquelle les pointillés indiquent la présence d'une double liaison.**

Ces composés sont préparés par déshydrogénation des composés correspondants de formule 1 dans lesquels les pointillés ne représentent rien.

Cette réaction de déshydrogénation est réalisée de façon connue en soi, par exemple par action de :

- soufre (cf. Organic Synthesis, vol. 2, edition John Wiley & Sons, 1988, page 423 ; Organic Synthesis, vol. 3, edition John Wiley & Sons, 1988, page 729) ;
- palladium sur charbon à 5% au reflux de la décaline (cf. Organic Synthesis, vol. 4, edition John Wiley & Sons, 1988, page 536) ;
- 2,3-dichloro-5,6-dicyano-1,4-benzoquinone ou DDQ (cf. Organic Synthesis, vol. 5, edition John Wiley & Sons, 1988, page 428 ; Synthesis, 1983, 310).

Les thiones de formules II et IIa sont des composés facilement préparés par synthèse organique à partir de produits commerciaux.

Les thiones de formule IIa sont des thiones de formule II dans lesquelles $R_4$ représente un atome d'hydrogène.

Ces composés peuvent notamment être préparés en suivant et éventuellement en adaptant l'un quelconque des procédés décrits dans :

- Spindler Juergen ; Kempter Gerhard ; Z. Chem. ; 27 ; 1. 1987 ; 36-37
  ou
- Setescak Linda L. ; Dekow Frederick W. ; Kitzen Jan M. ; Martin Lawrence L.; J. Med. Chem. ; 27 ; 3 ; 1984; 401-404.

Ces deux publications décrivent plus particulièrement la synthèse de la 1,3,4,5-tétrahydro-(1H,3H)-1,3-benzodiazépine-2-thione, de la 1,3,4,5-tétrahydro-(1H,3H)-4-phényl-1,3-benzodiazépine-2-thione et de la 1,3,4,5-tétrahydro-(1 H,3H)-3-méthyl-4-phényl-1,3-benzodiazépine-2-thione.

A titre d'exemple, lorsque $R_2$ représente aryle ou hétéroaryle éventuellement substitué et $R_3$ représente H, une voie de synthèse de la thione de formule II dans laquelle les pointillés ne représentent rien est proposée sur le schéma 1 ci-dessous.

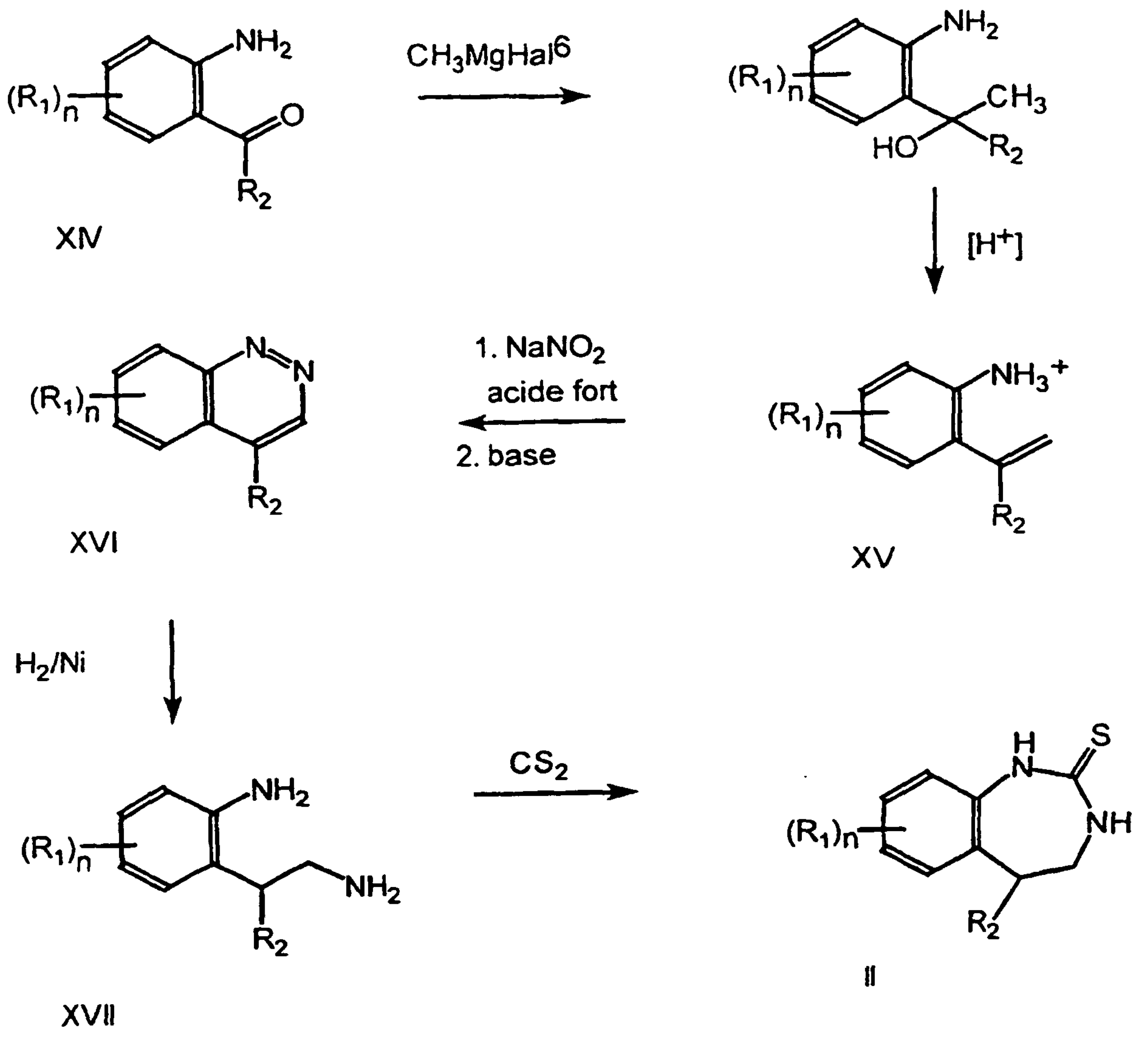


SCHEMA 1

La cétone XIV est traitée, dans les conditions usuelles, par un réactif de Grignard de formule $CH_3MgHal^6$ où $Hal^6$ est un atome d'halogène. On opère par exemple dans un éther, de préférence un éther aliphatique tel que l'éther de diéthyle ou de diisopropyle ou le tétrahydrofurane, à une température comprise entre 20 et 50°C, de préférence entre 30 et 40°C.

Après déshydratation de l'alcool intermédiaire (en milieu acide), on récupère le composé XV sous forme de sel. La nature du contre-ion, dans le composé XV (lequel contre-ion n'est pas représenté sur le schéma 1) dépend de l'acide utilisé pour la déshydratation. A l'étape suivante, on traite le composé XV par du nitrite de sodium en présence d'un acide fort tel que l'acide chlorhydrique, puis le composé intermédiaire est traité par une base et préférablement par un hydroxyde du type hydroxyde de métal alcalin ou hydroxyde d'ammonium.

Le composé diazo de formule XVI obtenu est ensuite soumis à une hydrogénation en présence de nickel dans un solvant de type polaire tel qu'un ($C_1$-$C_6$)alcanol ou un amide du type diméthylformamide, à une température comprise entre 30 et 100° C, de préférence à une température de 50 à 70° C.

La thione est finalement préparée en faisant réagir le composé hydrogéné XVII avec du disulfure de carbone, dans des conditions appropriées tel que par exemple au reflux d'un alcool aliphatique en $C_1$-$C_6$, par exemple au reflux de l'éthanol.

Un autre procédé de préparation de thiones de formule II dans laquelle $R_2$ et $R_3$ représentent tous deux un atome d'hydrogène et les pointillés ne représentent rien, est illustré sur le schéma 2 ci-dessous.

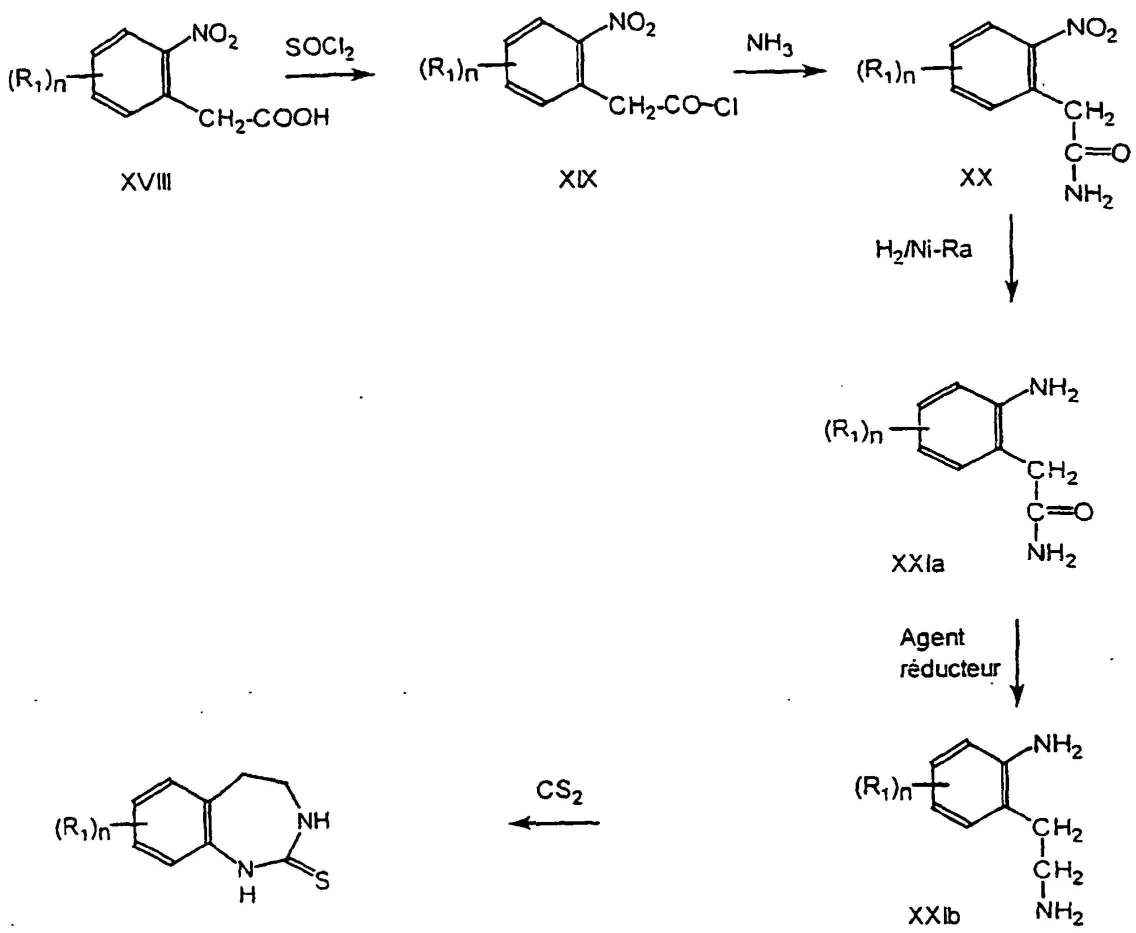


XVIII XIX XX XXIa XXIb II

SCHEMA 2

L'amine de formule XXIa est préparée de façon conventionnelle par action de chlorure de thionyle, puis d'ammoniac et enfin par hydrogénation catalytique en présence de nickel de Raney.

Puis, on procède à la réduction de la fonction carbonyle du composé XXIa par action d'un agent réducteur approprié. Des exemples de réducteurs appropriés sont les hydrures (tels que l'hydrure de lithium et aluminium, le borohydrure de sodium, le cyanoborohydrure de sodium, $BH_3/BF_3$-$Et_2O$ et $Et_3SiH$), le zinc en milieu acide chlorhydrique, le lithium en milieu ammoniacal ou le nickel de Raney en milieu éthanolique.

La réduction peut également être réalisée par hydrogénation catalytique, par exemple en présence de palladium sur charbon ou d'oxyde de platine.

On préfère opérer en présence de $AlLiH_4$. La réduction du composé XXIa conduit au composé XXIb.

On fait ensuite réagir l'amine XXIb avec du disulfure de carbone, de préférence dans un solvant polaire de type alcanol en $C_1$-$C_6$, (tel que par exemple l'éthanol) à une température entre 80 et 150° C en fin de réaction.

Les sulfures de formule Va sont facilement obtenus à partir des thiones correspondantes de formule II.

Une voie de synthèse possible consiste à faire réagir la thione de formule II appropriée :

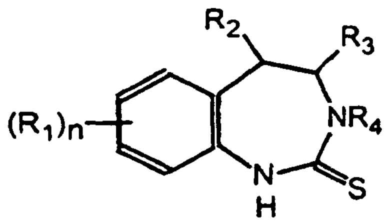

II

dans laquelle n, $R_1$, $R_2$, $R_3$ et $R_4$ sont tels que définis ci-dessus avec un halogénure $Hal^5$-alk dans lequel $Hal^5$ représente un atome d'halogène et alk représente ($C_1$-$C_6$)alkyle dans un solvant polaire protique tel qu'un alcool aliphatique, par exemple un ($C_1$-$C_6$)alcanol. Il est fortement souhaitable que la chaîne alkyle de l'alcool utilisé comme solvant corresponde exactement à la chaîne alk du dérivé halogéné.

La réaction de la thione II sur ce dérivé halogéné est préférablement conduite à une température comprise entre 15 et 50° C, de préférence entre 20 et 30° C, par exemple à température ambiante.

Ce procédé est particulièrement avantageux pour la préparation de composés de formule Va dans lesquels alk représente méthyle.

De manière préférée, $Hal^5$ représente un atome d'iode.

Les composés de formule XI dans laquelle $R_3$ représente un atome d'hydrogène peuvent être préparés en utilisant le procédé illustré sur le schéma 3 ci-dessous.

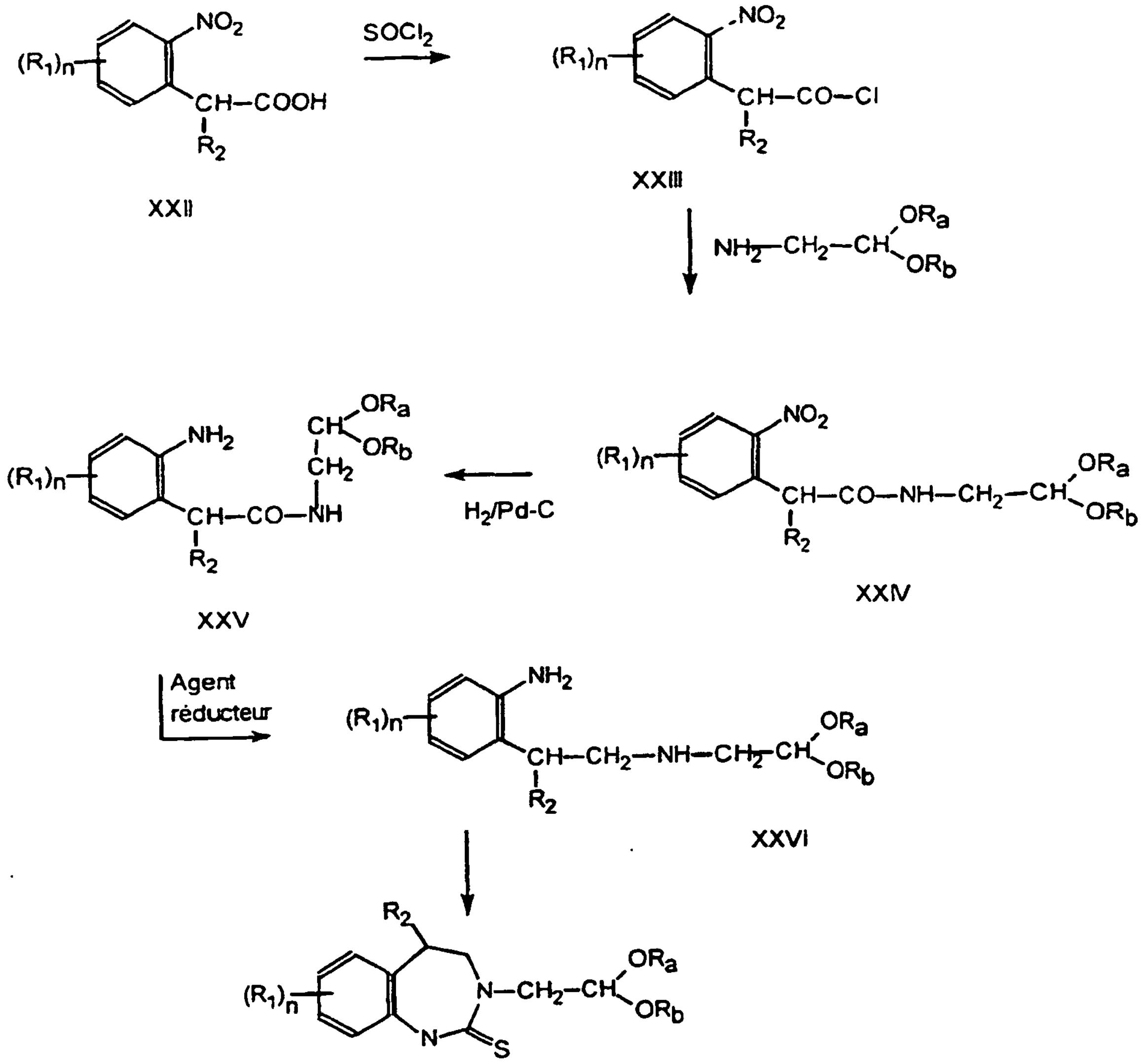


XXII XXIII XXIV XXV XXVI XI

SCHEMA 3

L'amide de formule XXIV est préparé de façon conventionnelle au départ de l'acide de formule XXII par action de chlorure de thionyle et de l'amine appropriée de formule $NH_2$-$CH_2$-$CH(OR_a)(OR_b)$ dans laquelle $R_a$ et $R_b$ sont tels que définis ci-dessus pour la formule XI.

Puis l'amide XXIV est soumis à une réaction d'hydrogénation en présence de palladium sur charbon de façon à convertir la fonction nitro en fonction amine. Cette transformation est réalisée dans les conditions classiques de la chimie organique.

La fonction carbonyle de l'amine résultante est alors réduite par action d'un hydrure approprié par exemple l'hydrure de lithium et aluminium, le borohydrure de sodium, le cyanoborohydrure de sodium ou l'hydrure de diisobutylaluminium.

Puis l'amine obtenue, XXVI, est traitée par du disulfure de carbone dans les mêmes conditions que décrit ci-dessus dans le cas du composé XVII (schéma 1) ou dans le cas du composé XXIb (schéma 2).

Les composés de formule XII dans laquelle $R_3$ représente l'hydrogène peuvent être synthétisés par mise en oeuvre du procédé illustré sur le schéma 4 suivant :

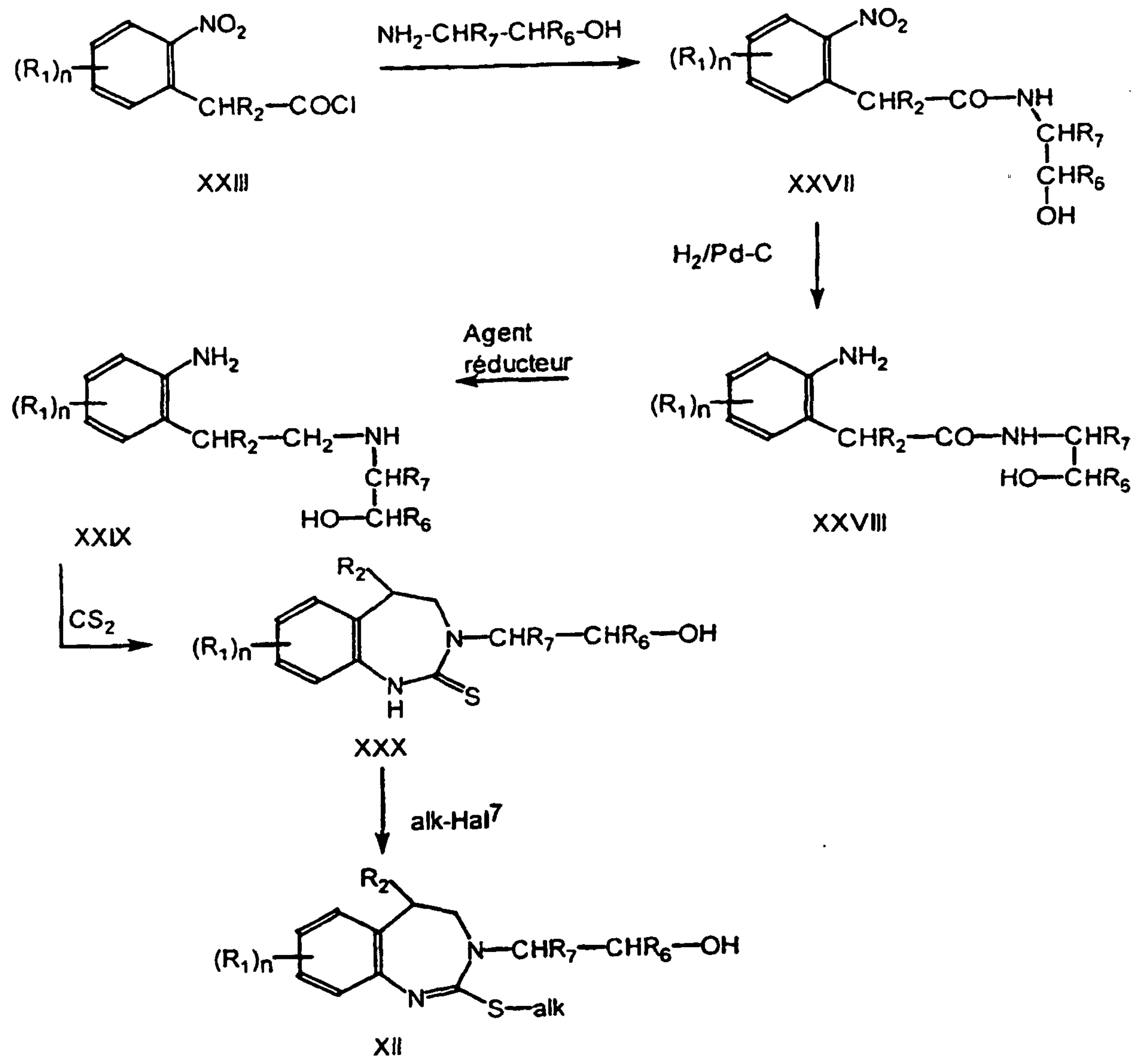


SCHEMA 4

L'amine de formule XXVII est préparée simplement en faisant réagir une amine de formule $NH_2$-$CHR_7$-$CHR_6$-OH dans laquelle $R_6$ et $R_7$ sont tels que définis ci-dessus pour la formule I avec le chlorure d'acide de formule XXIII. Cette réaction est mise en oeuvre dans les conditions classiques, de préférence en présence d'une base, et préférablement d'une base organique. Les trois étapes suivantes, qui conduisent au composé de formule XXX, sont mises en oeuvre dans des conditions comparables au cas de la transformation du composé XXIV en composé XI (schéma 3). Puis le composé XXX est mis à réagir avec $Hal^7$-alk dans laquelle $Hal^7$ représente un atome d'halogène et alk est ($C_1$-$C_6$)alkyle. Cette réaction pourra être mise en oeuvre dans les conditions précisées ci-dessus pour la transformation de la thione II en sulfure de formule Va. On préfère réaliser cette réaction dans un alcanol en $C_1$-$C_6$ dont la chaîne alkyle correspond exactement à la chaîne alk de alk-$Hal^7$ et avec un halogénure alk-$Hal^7$ dans lequel $Hal^7$ représente un atome d'iode.

L'activité hypolipidémiante des composés de l'invention résultent de leur aptitude à diminuer la sécrétion d'apo CIII. Le test biologique suivant a été mis au point de façon à mettre en évidence cette activité. Il révèle la capacité des composés de l'invention à diminuer la sécrétion d'Apo CIII par une lignée d'hépatocytes humains en culture Hep G2.

La lignée cellulaire Hep G2 provient d'un carcinome hépatique humain (ref. ECACC n° 85011430).

Les cellules sont mises en culture à 37°C, 5% $CO_2$ dans des plaques de microtitration de 96 puits à raison de 40 000 cellules (200 $\mu$l) par puits dans un tampon DMEM, 10% sérum de veau foetal, 1% Glutamax + antibiotiques pendant 24 heures. Le milieu de culture est ensuite retiré et remplacé par le même milieu contenant les substances à tester à une concentration de 10 $\mu$m. Les cellules sont incubées pendant 24 heures à 37°C, 5% $CO_2$ puis le milieu est

prélevé.

La quantité d'apolipoprotéine CIII sécrétée dans le milieu est mesurée à l'aide d'un dosage de type ELISA. Chaque échantillon de milieu de culture est dilué au 1/5 dans un tampon phosphate 100 mM, BSA 1%. 100 µl de chaque dilution sont déposés dans les puits de plaques de microtitration à 96 puits préalablement sensibilisés avec un anticorps polyclonal anti Apo CIII humain pendant 18 heures et passivés à raison de 1 µg par puits dans du PBS 100 mM et passivés avec 200 µl de PBS 100 mM, BSA 1% pendant 1 heure à 20°C.

Chaque dilution de milieu est incubée pendant 2 heures à 37°C, puis les puits sont lavés par 4 bains de PBS 100 mM, Tween 20 à 0,3%. 100 µl d'une solution diluée dans du PBS 100 mM, BSA 1% d'anticorps polyclonal anti Apo CIII couplé à la peroxydase sont ajoutés dans chaque puits et incubés à 37°C pendant 2 heures. Après un nouveau lavage identique au précédent, 100 µl d'un tampon phosphate 50 mM, citrate 15 mM, pH = 5,5 contenant 1,5 mg/ml d'orthophénylènediamine et 0,5 µl/ml de peroxyde d'hydrogène ($H_2O_2$) sont ajoutés dans chaque puits. La plaque est mise à incuber pendant 20 minutes à l'obscurité puis la réaction est arrêtée par ajout de 100 µl d'HCl 1N.

La densité optique est lue directement au spectrophotomètre à 492 nm. La quantité d'Apo CIII est calculée par rapport à une courbe d'étalonnage réalisée à partir d'un sérum humain titré en Apo CIII et dilué dans les mêmes conditions que l'Apo CIII contenue dans le milieu de culture.

En l'absence de traitement chimique, la réponse des cellules est de 100% (0% inhibition). Dans les conditions utilisées, l'effet du DMSO sur les cellules est négligeable. La toxicité des substances chimiques sur les cellules est mesurée par la technique de coloration au rouge neutre.

Les substances actives entraînent une diminution de la sécrétion d'Apo CIII dans le milieu par les cellules adhérentes. La concentration d'Apo CIII est mesurée pour chaque traitement et comparée au test témoin (pas de traitement).

Le pourcentage d'inhibition est calculé selon:

$$100 - \frac{\text{(concentration Apo CIII avec traitement x 100)}}{\text{concentration Apo CIII sans traitement}}$$

Le pourcentage d'inhibition n'est calculé que pour les substances ne présentant pas de toxicité sur les cellules Hep G2.

A titre d'exemple, le pourcentage d'inhibition mesuré pour le composé de formule I dans laquelle X = S; n = 0; $R_2 = R_3 = R_6 = H$; $R_7$ = 4-biphényle et $R_4$ et $R_5$ forment ensemble $-CR_6=CR_7$ (exemple 4 ci-après) est de 80% à 100 micromolaires. La concentration en composé de l'exemple 4 conduisant à une inhibition de 50% de la sécrétion d'Apo CIII dans ce test est de 17,4 µM. Aucune toxicité cellulaire n'est observée avec le composé de l'exemple 4 pour les concentrations étudiées.

L'invention est illustrée dans la suite à l'aide de préparations et d'exemples. Elle n'entend pas se limiter à la divulgation de ces exemples spécifiques.

**PREPARATION 1**

**Préparation de la thione de formule IIa dans laquelle n = 0 ; et $R_2 = R_3$=H**

Le composé du titre est préparé par mise en oeuvre du procédé décrit dans Spindler Juergen ; Kempter Gerhard ; Z. Chem. ; 27 ; 1 ; 1987 ; 36-37. Son point de fusion est de 195° C.

**PREPARATION 2**

**Préparation de la thione de formule IIa dans laquelle n = 0 ; $R_2 = -C_6H_5$ et $R_3$ = H**

Le composé du titre est préparé conformément à l'enseignement de FR 2 528 838.

**PREPARATION 3**

**Préparation de la thione de formule XI dans laquelle n = 0 ; $R_2 = R_3 = H$ ; $R_a = R_b = -CH_3$**

(a) N-(2,2-diméthoxyéthyl)-2-(2-nitrophényl)acétamide

On place dans un réacteur 21,0 g (0,2 mol) de l'acétal diméthylique de l'aminoacétaldéhyde en solution dans 200 ml de chloroforme ensemble avec 22,2 g (0,22 mol) de triéthylamine. Le milieu réactionnel est porté et maintenu à 10° C. Dans cette solution, on coule une solution de 0,2 mol de chlorure de l'acide 2-nitrophénylacétique dans 200 ml de chloroforme. On laisse le milieu réactionnel revenir à température ambiante et on poursuit l'agitation pendant 12 heures.

On ajoute alors une solution aqueuse de soude puis on laisse décanter la phase organique que l'on sépare et sèche sur sulfate de sodium anhydre. Après évaporation du solvant sous pression réduite, on obtient un solide beige que l'on recristallise dans un mélange d'hexane et d'acétate d'éthyle. On isole ainsi 35 g d'un solide présentant un point de fusion compris entre 89 et 90° C.

(b) N-(2,2-diméthoxyéthyl)-2-(2-arninophényl)acétamide.

On procède à l'hydrogénation de 40 g du composé obtenu à l'étape (a) ci-dessus en solution dans 750 ml d'éthanol dans un autoclave en présence de 5 g de palladium sur charbon à 5% sous une pression de 120 bars d'hydrogène.

Après filtration du catalyseur et évaporation du solvant, on obtient 35 g d'une huile utilisée à l'état brut dans la suite de la synthèse.

(c) N-(2,2-diméthoxyéthyl)-2-(2-aminophényl)éthylamine.

Dans un réacteur d'un litre maintenu sous atmosphère inerte, on place 28,1 g (0,74 mol) d'hydrure de lithium et aluminium en suspension dans 280 ml de tétrahydrofurane anhydre.

On refroidit le milieu réactionnel à une température inférieure à 10° C et on coule dans cette solution, maintenue à cette température, 35,3 g du composé obtenu à l'étape (b) dissous dans 350 ml de tétrahydrofurane anhydre. L'ensemble est porté sous agitation pendant 8 heures au reflux du solvant.

On refroidit à nouveau le milieu réactionnel à une température inférieure à 10° C et on coule lentement 100 ml d'eau dans cette solution afin de détruire l'excès d'hydrure présent. On essore les hydroxydes d'aluminium formés et on les rince au chloroforme.

Les phases organiques séparées sont séchées sur sulfate de sodium anhydre puis évaporées sous pression réduite. On isole ainsi 23 g d'une huile que l'on utilise à l'état brut à l'étape suivante.

(d) 3-(2,2-diméthoxyéthyl)-4,5-dihydro-(1H,3H)-1,3-benzodiazépine-2-thione.

Dans un réacteur de 500 ml, on place 22,6 g (0,298 mol) de sulfure de carbone en solution dans 180 ml d'éthanol.

Dans cette solution, on coule, à température ambiante, 0,149 mol du composé obtenu à l'étape précédente en solution dans 150 ml d'éthanol. La température s'élève de 18 à 22° C. On laisse le milieu réactionnel sous agitation pendant 12 heures à la température ambiante puis on porte le milieu réactionnel 6 heures au reflux du solvant. On laisse ensuite revenir à température ambiante puis on évapore sous pression réduite le solvant. On obtient une huile épaisse verte que l'on recristallise dans 100 ml d'éthanol. On isole ainsi 21 g d'un solide présentant un point de fusion de 79 à 81 ° C.

**PREPARATION 4**

**Préparation de la thione de formule XXX dans laquelle n = 0 ; $R_2 = R_6 = H$ ; $R_7 = -C_6H_5$**

Le composé du titre est préparé conformément à l'enseignement de FR 2 518 544.

**PREPARATION 5**

**Préparation du composé de formule XIII dans lequel n = 0 ; $R_2 = R_3 = R_6 = H$ : $R_7 = -C_6H_5$**

Dans un réacteur de 250 ml, on place 11,6 g (0,039 mol) de 3-(2-hydroxy-1 phényléthyl)-(1H,3H)-1,3-benzodiazépine-2-thione en suspension dans 120 ml d'éthanol.

A cette solution an ajoute 11,0 g (0,078 mol) d'iodure de méthyle puis on porte le milieu réactionnel 1 heure au reflux du solvant. On observe un dégagement important de méthylmercaptan.

On laisse l'ensemble revenir à la température ambiante puis on évapore le solvant sous pression réduite. On reprend le résidu avec de l'éther diéthylique et une solution aqueuse diluée d'hydroxyde d'ammonium. Il se forme un précipité blanc que l'on isole par essorage. On obtient 7,6 g d'un produit présentant un point de fusion de 137 à 139° C que l'on recristallise dans un mélange d'hexane et d'acétate d'éthyle. Le produit ainsi isolé présente un point de fusion de 142 à 144° C.

Le chlorhydrate du composé du titre recristallise dans l'acétone et présente un point de fusion de 132 à 135° C.

**PREPARATION 6**

**Préparation du sulfure de formule V dans laquelle n = 0 ; $R_2 = R_3$ = H et alk = $-CH_3$**

Dans un réacteur de 1 l, on charge 33,2 g (0,1862 mole) de la thione obtenue à la préparation 1 et 300 ml de méthanol. Le mélange est agité jusqu'à dissolution complète. Puis on coule, goutte à goutte, dans ce mélange 23,2 ml (0,3724 mol, 2 éq.) de $CH_3I$ en solution dans 50 ml de méthanol.

On porte au reflux le milieu réactionnel. Après 1 h, le solvant est évaporé sous pression réduite puis le résidu est repris dans 500 ml d'éther diéthylique. Un précipité se forme, lequel est dissous et lavé trois fois avec 50 ml d'éther diéthylique puis séché sous pression réduite. On isole ainsi 59,3 g d'un produit couleur crème (rendement = 99,4 %) présentant un point de fusion de 171-173° C.
RMN $^1H$ (300 MHz, DMSO) δ (ppm) :
11,42 (1H, s); 10,10 (1H, s) ; 7,45-7,24 (4H, m) ; 3,80-3,77 (2H, m); 3,27-3,24 (2H, m) ; 2,85 (3H, s).

**EXEMPLE** 1

**Préparation du composé de formule I dans laquelle X = $-NCH_3$; n = 0 ; $R_2 = R_3 = R_6$ = H ; $R_4 + R_5 = -CR_6{=}CR_7-$ ; $R_7$ = -OH**

Dans un réacteur de 250 ml maintenu sous atmosphère d'azote, on charge 8,5 g (0,0264 mol) du sulfure de formule V obtenu à la préparation 6, 125 ml d'acétonitrile séché sur tamis moléculaire (4 Å) et 6,8 g de sarcosinate d'éthyle. On laisse l'ensemble sous agitation à température ambiante pendant 15 h, puis on additionne à nouveau 2 g de sarcosinate d'éthyle et on porte le milieu réactionnel 6 h au reflux. Ensuite, on ajoute à nouveau au milieu réactionnel 2 g de sarcosinate d'éthyle et on maintient l'ensemble à nouveau 14 h au reflux. Après ce temps de réaction, le dégagement de $CH_3SH$ n'est plus observé.

On concentre alors le milieu réactionnel par évaporation sous pression réduite, puis on reprend le solide beige obtenu dans 200 ml d'eau plus 30 ml d'une solution aqueuse de bicarbonate de sodium à 7%. On extrait la solution au dichlorométhane, sèche sur sulfate de sodium anhydre, puis évapore les solvants. Le résidu est alors purifié par chromatographie sur gel de silice en utilisant un mélange dichlorométhane/acétate d'éthyle:4/1. On isole ainsi 3,4 g d'un solide jaune d'un point de fusion de 132-134° C. Après recristallisation dans un mélange de 30 ml d'hexane et de 40 ml d'acétate d'éthyle, on isole 2,7 g d'un solide jaune clair (rendement = 47,5%) d'un point de fusion de 132-134° C.
RMN $^1H$ (300 MHz, DMSO) δ (ppm) :
7,30-7,27 (1H, m) ; 7,22-7,16 (2H, m) ; 7,05-6,99 (1H, m) ; 4,18 (2H, s) ; 3,88 (2H, s) ; 3,14 (3H, s) ; 3,12-3,07 (2H, s).

**EXEMPLE 2**

**Préparation du composé de formule I dans laquelle X = -NH ; n = 0 ; $R_2 = R_3 = R_6$ = H ; $R_4 + R_5 = -CR_6{=}CR_7-$ ; $R_7 = -C_6H_5$**

**a) Préparation du composé de formule VII dans laquelle $R_6 = R_2 = R_3$ = H ; n = 0 ; $R_7 = -C_6H_5$ ; $R_a$ et $R_b$ forment ensemble $-CH_2-CH_2-$**

Dans un réacteur de 100 ml maintenu sous atmosphère d'azote, on charge 4,4 g (0,01381 mol) du sulfure obtenu à la préparation 6, 80 ml d'acétonitrile et 5,2 g (0,029 mol ; 2,1 éq.) de l'amine suivante :

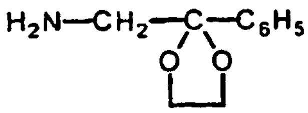

On porte l'ensemble 12 h à 50° C, puis on laisse revenir le milieu réactionnel à température ambiante (20° C). On additionne alors 100 ml d'éther diéthylique. Le précipité formé est filtré à 20° C et lavé 3 fois avec 15 ml d'éther diéthylique puis séché sous pression réduite. On obtient ainsi 5,5 g d'un produit solide couleur crème d'un point de fusion de 220°C. Le résidu est repris avec une solution aqueuse de bicarbonate de sodium à 7% (100 ml) et laissé 30 mn sous agitation, puis filtré, lavé à l'eau et séché sous pression réduite.

On obtient ainsi 4,5 g d'un solide couleur crème présentant un point de fusion de 217-219°C. Après recristallisation dans 100 ml d'éthanol, on isole 4,3 g d'un solide de couleur blanche d'un point de fusion de 217-219° C. Ce composé est le sel iodhydrate du composé du titre, ainsi qu'il résulte de l'analyse centésimale du produit obtenu (rendement = 69 %).
RMN $^1$H (300 MHz, DMSO) δ (ppm) :
9,39 (s, 1H) ; 8,36 (1H, s) ; 7,32-7,00 (7H, m) ; 6,87 (2H, t, J = 7 Hz) ; 3,9-3,88 (2H, m) : 3,62-3,60 (2H, m) ; 3,48 (2H, s) ; 3,26 (2H, t, J = 4,5 Hz) ; 2,80 (2H, t, J = 4,6 Hz).

**b) Préparation du composé de formule 1 dans laquelle X = NH ; n = 0 ; $R_2$ = $R_3$ = $R_6$ = H ; $R_4$ + $R_5$ = -$CR_6$=$CR_7$- ; $R_7$ = -$C_6H_5$**

Dans un réacteur de 500 ml maintenu sous atmosphère azote, on charge 3 g du composé obtenu à l'étape a) ci-dessus (0,009277 mol), 200 ml d'éthanol et 200 ml d'HCl 5N. On porte l'ensemble au reflux pendant 5 h puis le solvant est évaporé sous pression réduite. On additionne au résidu 200 ml d'eau, et on lave 2 fois avec 150 ml d'éther diéthylique. La solution est basifiée avec une solution aqueuse d'hydroxyde de sodium à 30 % en maintenant la température au-dessous de 20° C. Le précipité crème formé est filtré puis lavé à l'eau et séché sous pression réduite à 80° C. On isole ainsi 1,8 g (rendement = 73,8%) d'un solide de couleur crème présentant un point de fusion de 194-206° C.
RMN $^1$H (300 MHz, DMSO) δ (ppm) :
9,18 (1H, s) ; 7,28-7,11 (8H, m) ; 6,67-6,65 (1H, m) : 6.55 (1H, s) ; 3,94 (2H, t, J = 4,7 Hz) ; 2,91 (2H, t, J = 4,6 Hz).

**EXEMPLE 3**

**Préparation du composé de formule 1 dans laquelle X = $NCH_3$ ; n = 0 ; $R_2$= $R_3$ = $R_6$ = H ; $R_4$ + $R_5$ = -$CR_6$=$CR_7$ ; $R_7$ = -$C_6H_5$**

Dans un réacteur de 100 ml, on charge 1,4 g (0,00531 mol) du composé obtenu à l'exemple 2, 46 ml de diméthylformamide séché sur tamis moléculaire (4 Å). L'ensemble est agité jusqu'à dissolution complète. On additionne alors, à 20° C, 0,22 g d'une dispersion d'hydrure de sodium à 60% dans l'huile (0,005575 ; 1,05 éq.) et on laisse l'ensemble réagir, sous agitation pendant 30 mn. Ensuite on additionne en une seule fois 0,4 ml (0,006372 ; 1,2 éq.) d'iodure de méthyle. On laisse l'ensemble 48 h sous agitation puis on verse le milieu réactionnel dans 600 ml d'eau et on extrait la solution au dichlorométhane. Les extraits réunis sont lavés à l'eau, séchés sur sulfate de sodium anhydre, et le solvant est évaporé sous pression réduite. On obtient 1.1 g d'une huile jaune. Le sel maléate de ce composé est préparé par action d'un équivalent d'acide maléique dans le méthanol à température ambiante. Le solvant est évaporé et le résidu est recristallisé dans le méthanol. On isole ainsi 0,78 g (rendement = 37,5 %) d'un solide blanc d'un point de fusion de 173-175° C.
RMN $^1$H (300 MHz, DMSO) δ (ppm) :
7,51-7,23 (10H, m) ; 6,10 (2H, s) ; 4,11-4,08 (2H, m) ; 3,54 (3H, s) ; 3,19 (2H, t, J = 5,1 Hz).

**EXEMPLE 4**

**Préparation d'un composé de formule 1 dans laquelle X = S ; n = 0 ; $R_2$ = $R_3$ = $R_6$ = H ; $R_7$ = p-(phényl)phényle ; $R_4$ et $R_5$ forment ensemble -$CR_6$=$CR_7$-**

Dans un réacteur de 500 ml muni d'un réfrigérant, on introduit 16,5 g (92,5 mmol) de la thione obtenue à la préparation 1, 390 ml d'acide acétique glacial et 25,5 g (92,5 mmol) de (bromométhyl)(para-phénylphényl)cétone, l'ensemble est porté progressivement au reflux, sous agitation, et maintenu 3 h au reflux. Le milieu réactionnel est alors

refroidi à 15° C. Le précipité est filtré (bromhydrate), rincé à l'éther diéthylique et séché. Le résidu est repris dans 200 ml d'eau glacée et la solution résultante est basifiée lentement par addition d'une solution aqueuse de soude à 30% sous agitation vigoureuse. On additionne ainsi la quantité nécessaire de soude pour observer la stabilité du pH alcalin. La solution est ensuite extraite 2 fois au chlorure de méthylène. Puis les extraits sont rincés à l'eau et séchés sur sulfate de sodium anhydre puis le solvant est évaporé sous pression réduite. On isole ainsi un solide jaune pâle (rendement = 85 %) qui est recristallisé dans le toluène de façon à obtenir le composé du titre, sous forme pure, lequel présente un point de fusion de 199,5-200° C (exemple 4).

Le sel chlorhydrate de ce composé est préparé par addition d'une solution à 33% d'acide chlorhydrique dans l'éthanol. Le point de fusion de ce sel est de 299,5-300°C (exemple 44).

RMN $^1$H (300 MHz, DMSO-d6) :

3,02 (2H,m) ; 3,97 (2H, m) ; 6,4 (1H, s) ; 6,8 (1H, m) ; 7 (2H, m) ; 7,1 (1H,m) ; 7,4-7,6 (5H, m) ; 7,7-7,9 (4H, m).

RMN $^1$H (300 MHz, DMSO-d6) du chlofiydrate :

3 (2H, m) ; 4 (2H, m) ; 6,8-7,7 (14H) ; 13 (1 H, s échangeable).

**EXEMPLE 6**

**Préparation d'un composé de formule I dans laquelle X = S ; n = 0 ; $R_4$ et $R_5$ forment ensemble $-CR_6=CR_7-$; $R_2 = -C_8H_5$; $R_3$ = H; $R_6$ = H; $R_7$ = -OH**

Dans un réacteur de 250 ml contenant 125 ml d'acide acétique, on introduit 10 g (0,039 mol) de la thione obtenue à la préparation 2. On coule goutte à goutte dans cette solution, 7,9 g (0,047 mol) de bromoacétate d'éthyle et on porte le milieu réactionnel 9 heures à reflux. Il se forme un précipité blanc. Après retour à la température ambiante, on essore le bromhydrate formé. On sèche le produit et on le met en suspension dans l'eau. On additionne à cette suspension une solution d'hydroxyde d'ammonium à 30 % jusqu'à pH basique. On essore puis sèche le produit avant d'effectuer une recristallisation dans un mélangé d'hexane et d'acétate d'éthyle. On isole ainsi 8,2 g du composé du titre, lequel présente un point de fusion de 156-158° C.

**EXEMPLE 7**

**Préparation d'un composé de formule I dans laquelle X = S ; n = 0 ; $R_4$ et $R_5$ forment ensemble $-CR_6=CR_7-$; $R_2 = R_3 = R_6$ = H; $R_7$ = OH**

On place dans un tricol de 250 ml, 3,0 g (0,0168 mol) de 2-thione-4,5-dihydro-1,3-benzodiazépine et 3,75 ml (0,0336 mol) de bromoacétate d'éthyle dans 50 ml de toluène.

On porte ensuite le mélange réactionnel 1 heure au reflux sous agitation. On laisse revenir l'ensemble à température ambiante puis on ajoute de l'eau et une solution aqueuse d'hydroxyde d'ammonium au milieu réactionnel et on extrait par de l'acétate d'éthyle. Après séchage des différents extraits organiques sur sulfate de sodium anhydre, on évapore le milieu réactionnel. On isole ainsi 1,4 g d'un solide ocre qui recristallise dans l'éthanol. Après recristallisation, le point de fusion de ce solide est de 111 à 112° C.

RMN$^1$H(300 MHz, $CDCl_3$) $\delta$(ppm) : 3,23-3,25 (2H,m); 4,18(4H,s); 7,26-7,3(2H,m); 7,43-7,5(2H,m).

**EXEMPLE 8**

**Préparation d'un composé de formule I dans laquelle X = S ; n = 0 ; $R_4$ et $R_5$ forment ensemble $-CR_6=CR7-$; $R_2 = R_3 = R_6 = R_7$ = H**

Dans un réacteur de 250 ml on introduit 14,0 g du composé obtenu à la préparation 3 dans 140 ml d'une solution aqueuse d'acide sulfurique à 50%. On porte l'ensemble 2 heures au reflux du solvant. On laisse le milieu réactionnel revenir à la température ambiante puis on jette le milieu réactionnel sur un mélange d'eau et de glace. Après extraction au chloroforme et séchage des extraits sur sulfate de sodium anhydre on évapore le solvant. On obtient ainsi 9 g d'une huile épaisse. Cette huile est dissoute dans 100 ml d'acétone. On ajoute alors 5,7 g d'acide maléique. Le produit obtenu après concentration de la solution est la maléate du composé du titre. Celui-ci est recristallisé dans l'acétone. Le produit obtenu présente un point de fusion compris entre 121 et 123° C.

Les composés des exemples suivants 9 à 192 ont été obtenus en utilisant les procédés illustrés dans les exemples 1 à 8 précédents.

Les tableaux 3 à 5 suivants rapportent les données de caractérisation obtenues pour chacun de ces composés.

F désigne le point de fusion.

Les spectres RMN ont été enregistrés à 300 MHz dans le solvant S.

Les abréviations s, d, t et m ont les significations suivantes :

- s : singulet
- d : doublet
- t : triplet
- m : massif.

Tableau 3

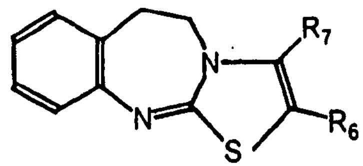

| Ex | $R_6$ | $R_7$ | F(°C) | RMN$^1$H : δ(ppm) |
|---|---|---|---|---|
| 34 | $-CH_3$ | $C_6H_5$ | 112-113 | S = DMSO-d6 2,1(3H,s);3,1(2H,m);3,9(2H,m);7-7,3(4H,m);7,6-7,7(5H,m). |
| 35 | -H | -CO-Oet | 140-150 | S= DMSO-d6 1,5(3H,t,J=7,1Hz);3,5(2H,m);4,5(2H,d,7,1Hz);4,9(2H,m);7,3-7,6(4H,m); 8,2(1H,s). |
| 36 | -H | 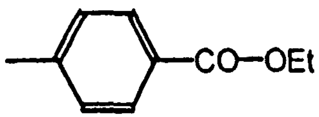 | 231-233 | S = DMSO-d6 1,3(3H,t,J=7,1Hz);4,3(2H,d,J=7,1Hz); 3,3(2H,m);4,2(2H,m);7,2-7,4(5H,m); 7,7(2H,d,J=8,2Hz) ; 8,1(2H,d,J=8,2Hz). |
| 37 | -H | -$^t$Bu | 112,3 | S = DMSO-d6 1,26(9H,s);3,1(2H,m);4,1(2H,m);1,95(1H,s);6,7-7(4H,m). |

| | | | | |
|---|---|---|---|---|
| 38 | -H | 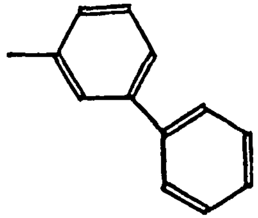 | 138-139 | S = DMSO-d6<br>3(2H,m) ; 4(2H,m) ; 6,5(1H,s);6,8(1H,t,J=7,3Hz); 6,9(2H,m) ; 7,1(1H,t,J=7,3Hz) ; 7,4-7,6(5H,m) ; 7,7(4H,m). |
| 39 | -H | 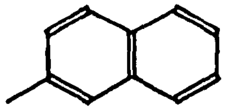 | 188,5-189,5 | S = DMSO-d6<br>6,6(1H,s);6,9-7,3(4H,m) ; 7,7(3H,m) ; 8,1(4H,m) ; 3,1(2H,m) ; 4,1(2H,m). |
| 40 | H | 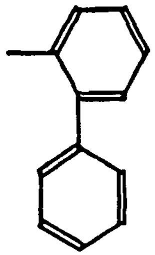 | 198,5-199 | S = $CDCl_3$<br>2(1H,m);2,6(1H,m);3,4(2H,m);6(1H,s); 6,7(2H,m);7,1(2H,m);7,3-7,6(9H,m). |
| 41 | -H | 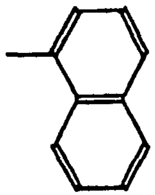 | 209,3 | S = DMSO-d6<br>2,9(2H,m);3,5(2H,m);6,4(1H,s);6,7(1H,m);6,8(1H,m);7(1H,m);7,1(1H,m);7,6(4H,m);7,7(1H,m);8(2H,m). |
| 42 | $-C_6H_5$ | H | 199,5-200 | S = DMSO-d6<br>3(2H,m);4(2H,m);6,8-7,3(10H,m). |

| | | | | |
|---|---|---|---|---|
| 43 | -H | 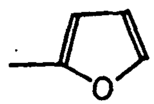 | 157-158 | S = DMSO-d6 3,1(2H,m);4,2(2H,m);6,5(1H);6,8(1H,s);6,9-7,3(5H,m);7,7(4H,s);13,5(1H échangeable,s). |
| 44 HCl | -H | 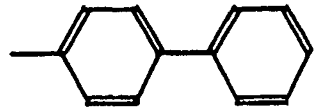 | 299,5-300 | --- |
| 45 | $-CH_2-COOH$ | $-C_6H_5$ | 216 | --- |
| 46 | -H | $-C_6H_5$ | 223-231 | S=DMSO-d6 3,4-3,5(2H,m);4,3-4,4(2H,m);7,3-7,5(4H,m);7,6-7,7(6H,m); 14 (1H,s,échangeable avec $CF_3COOD$). |
| 47 | -H | 2-naphtyle | 188,5-189,5 | ---------- |
| 48 | $-CH_3$ | $-C_6H_5$ | 112-113 | |
| 49 | -H | 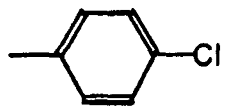 | 154°C | S = $CDCl_3$ 3,04-3,08(2H,m); 3,9-3,93(2H,m);5,94 (1H,s); 6,9-6,95(2H,m); 7,18-7,29(4H,m); 7,41- 7,44(2H,m). |
| 50 | -H | $-CH_3$ | 194-196 | S=DMSO-d6 0,7(3H,s);3,3-3,4(2H,m);4,3-4,4(2H,m);7,0(1H,s); 7,1-7,2(1H,m);7,3-7,4(2H,m);7,50-7,55(1H,m). |
| 51 | -H | $-CH_2-C_6H_5$ | 130-132 | S=$CDCl_3$ 2,8-2,9(2H,m);3,7(2H,s);3,7-3,5(2H,m);5,6(1H,s); 6,7-6,8(2H,m);7,0-7,3(7H,m). |

| | | | | |
|---|---|---|---|---|
| 52 | -H | 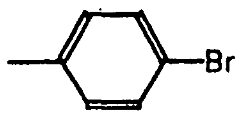 | 154°C | S=$CDCl_3$ 3,26-3,29(2H,m);4,12-4,15(2H,m);6,15(1H,s); 7,11-7,18(2H,m) ;7,36-7,5(4H,m) ; 7,78-7,8 (2H, m) |
| 53 | -H | 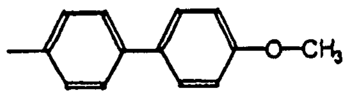 | 189°C | S=$CDCl_3$ 3,04-3,07(2H,m);3,85(3H,s);3,96-4(2H,m) ; 5,92(1H,s) ; 6,86-7,6(12H,m). |
| 54 | -H | 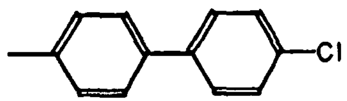 | 226-228 | S=DMSO-d6 3,0(2H,m);3,9(2H,m);6,4(1H,s);6,8-7,0(4H,m) ; 7,5-7,6(4H,m) ; 7,7-7,8(4H,m). |
| 55 | -H | 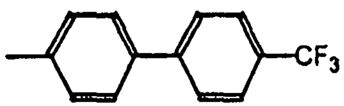 | 201°C | S=$CDCl_3$ 2,88-2,91(2H,m);3,79-3,82(2H,m) ; 5,79(1H,s);6,68-6,77(2H,m);6,96-7,02(2H,m);7,07-7,26(2H,m);7,46-7,56(6H,m). |
| 56 | -H | 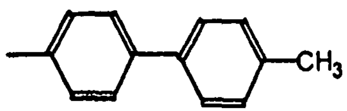 | 213 | S=$CDCl_3$ 2,58(3H,s);3,24(2H,t;J=4,6Hz);4,16(2H,t; J =4,6Hz) ; 6,11(1H,s); 7,02-7,12(2H,m); 7,31-7,81(10H,m). |
| 57 | -H | 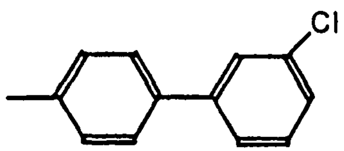 | 174-175 | S=$CDCl_3$ 3,23(2H,q;J=2,33Hz) ; 4,14(2H,q;J=2,33Hz) ; 6,14(1H,s); 7,07-7,09(2H,m) ; 7,34-7,79(10H,m). |

| | | | | |
|---|---|---|---|---|
| 58 | -H | 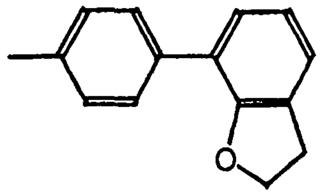 | 173 | S=$CDCl_3$<br>3,08(2H,t;J=4,6Hz);3,32(2H,t;J=8,75Hz)<br>;<br>4,02(2H,t;J=4,6Hz);4,67(2H,t;J=8,78Hz);<br>5,96(1H,s);6,88-7,01(3H,m);7,2-<br>7,4(6H,m);7,77-7,86(2H,m). |
| 59 | -H | 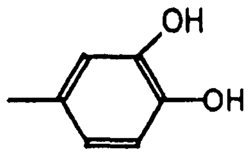 | 183-185 | S=DMSO-d6<br>3,11-3,12(2H,m);3,98-<br>4,01(2H,m);6,28(1H,s);6,81-<br>7,23(7H,m);9,4(2H,s;échangeable<br>$CF_3COOD$). |
| 60 | -H | 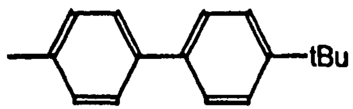 | 176 | S=$CDCl_3$<br>1,52(9H,s);3,21(2H,q;J=2,3Hz);4,14(2H,<br>q;J=2,3Hz) ; 6,09(1H,s);7-7,07(2H,m);7-<br>32-7,8(10H,m). |
| 61 | -H | 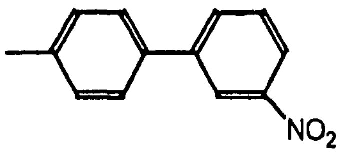 | 120-123 | S = $CDCl_3$<br>3,15(2H,t;J=4,65Hz);4,07(2H,t;J=4,65<br>Hz) ; 6,08(1H,s) ; 6,96-8,54(12H,m). |

Le tableau 4 suivant illustre en outre la préparation de composés répondant à la formule suivante :

Tableau 4

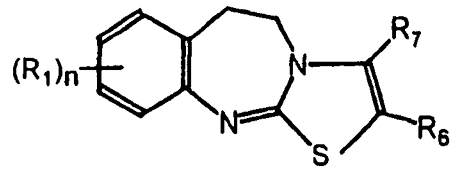

| Ex | n/R₁ | $R_6$ | $R_7$ | F(°C) | RMN$^1$H:δ(ppm) |
|---|---|---|---|---|---|
| 62 | O/- | $CH_2$-CO-OEt | -$C_6H_5$ | 192-193 | - |
| 63 | O/- | -H | -$CH_2$-$CH_2$-$NEt_2$ | 104-106 | - |
| 64 | O/- | 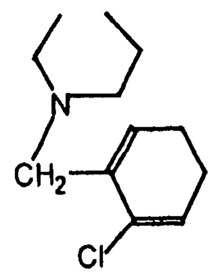 | | 223-225 | - |

| | | | | | |
|---|---|---|---|---|---|
| 65 | O/- | 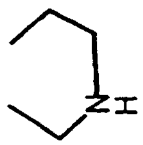 | | 299-301 | ---------- |
| 66 | 1/7-Cl | -H | 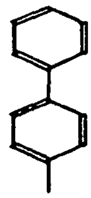 | 190-192 | S=$CDCl_3$<br>3,0-2,9(2H,m);<br>3,9-3,8 (2H,m);<br>5,9(1H,s);6,8(1H,m);<br>7,1-6,9(2H,m);7,6-7,3(9H,m). |

**EXEMPLE 66**

En utilisant les procédés illustrés dans les exemples précédents, on prépare le composé de formule :

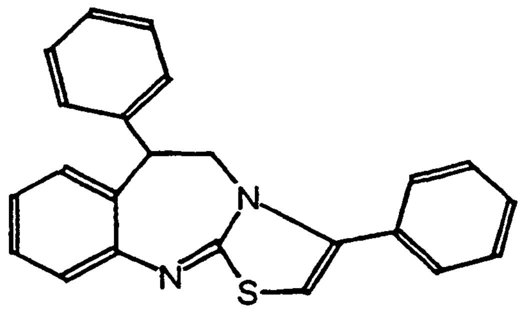

présentant un point de fusion de 184-185° C.

L'invention concerne en outre les compositions pharmaceutiques contenant une quantité efficace d'au moins un composé de formule I tel que défini ci-dessus en association avec au moins un véhicule pharmaceutiquement acceptable.

Selon un autre de ses aspects, l'invention concerne l'utilisation d'un composé de formule I tel que défini ci-dessus pour la préparation d'un médicament destiné à prévenir ou traiter les dyslipidémies, l'athérosclérose, le diabète ou ses complications.

TABLEAU 5

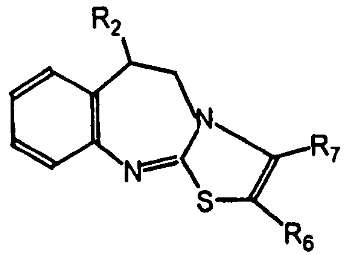

| Exemple | $R_2$ | $R_7$ | $R_6$ | Données de caractérisation |
|---|---|---|---|---|
| 67 | H | 4-phénoxyphényle | H | F = 204 - 205 °C<br>RMN 300 MHz (DMSO) : 3,2 (2 H, m) ; 4,1 (2 H, m) ; 6,3 (1 H, s) ; 6,8 - 6,9 (2 H, m) ; 6,9 - 7,0 (2 H, m) ; 7,1 - 7,2 (4 H, m) ; 7,2 - 7,3 (1 H, m) ; 7,4 - 7,5 (4 H, m)<br>MS : 371,3 (ES+) |
| 68, HCl | H | 4-(p-chlorobenzoyl)phényle | H | F = 253 - 255 °C<br>RMN 300 MHz (DMSO) : 3,2 (2 H, m) ; 4,2 (2 H, m) ; 7,1 - 7,5 (5 H, m) ; 7,68 (2 H, d, J = 8,7 Hz) ; 7,73 (2 H, d, J = 8,7 Hz) ; 7,81 (2 H, d, J = 8,7 Hz) ; 7,89 (2 H, d, J = 8,7 Hz) ; 12,9 (1 H, s)<br>MS : 417,3 (ES+) |
| 69, HCl | H | 3,4-éthylènedioxyphényle | H | RMN 300 MHz (DMSO) : 3,2 - 3,3 (2 H, m) ; 4,1 - 4,2 (2 H, m) ; 4,3 (4 H, s) ; 6,9 - 7,0 (3 H, m) ; 7,1 - 7,2 (2 H, m) ; 7,2 - 7,3 (1 H, m) ; 7,3 - 7,4 (2 H, m) ; 13,0 (1 H, s)<br>MS : 337,3 (ES+) |

| | | | | |
|---|---|---|---|---|
| 70, HCl | H | 4-cyanophényle | H | MS : 304 (ES+) |
| 71, HCl | H | 3,4-méthylènedioxyphényle | H | MS : 322,8 (ES+) |
| 72, HCl | H | 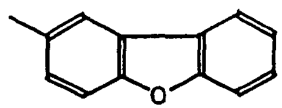 | H | MS : 368,8 (ES+) |
| 73, HCl | H | 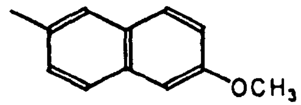 | H | MS : 358,8 (ES+) |
| 74, HCl | H | 3,4,5-triméthoxyphényle | H | MS : 369 (ES+) |
| 75, HCl | H | 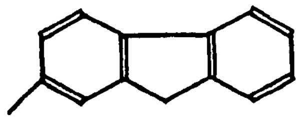 | H | MS : 367 (ES+) |
| 76, HCl | H | 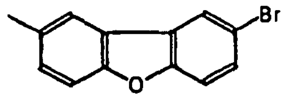 | H | MS : 447 et 449 (ES+) |
| 77, HCl | H | 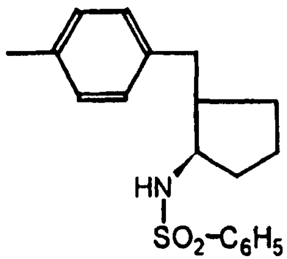 | H | MS : 516 (ES+) |
| 78, HCl | H | 3-cyanophényle | H | MS : 304 (ES+) |

| | | | | |
|---|---|---|---|---|
| 79, HCl | H | benzothién-3-yle | H | MS : 335 (ES+) |
| 80, HCl | H | 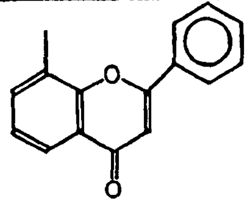 | H | MS : 440,8 (ES+) (+$H_2O$) ; 438,8 (ES-) (+$H_2O$) |
| 81, HCl | H | 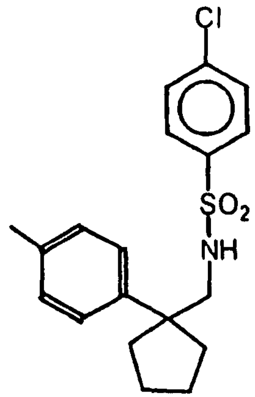 | H | MS : 550, 551, 552 et 553 (ES+) |
| 82, HCl | H | 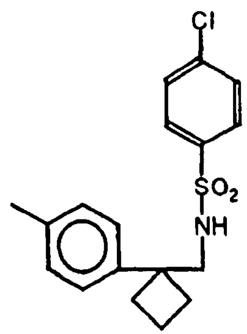 | H | MS : 536, 537, 538 et 539 (ES+) ; 534, 535, 536 et 537 (ES-) |

| | | | | |
|---|---|---|---|---|
| 83, HCl | H | 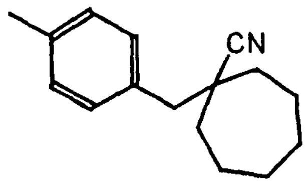 | H | MS : 414 (ES+) |
| 84, HCl | H | 4-carboxyméthyl | H | MS : 337 (ES+) |
| 85, HCl | H | 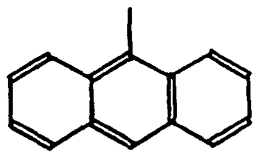 | H | MS : 379 (ES+) |
| 86, HCl | H | 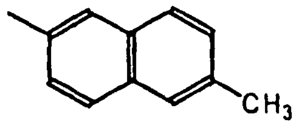 | H | MS : 343,2 (ES+) |
| 87, HCl | H | 4-méthylthiophényle | H | MS : 325,2 (ES+) |
| 88, HCl | H | 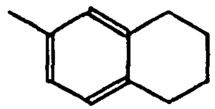 | H | MS : 333,2 (ES+) |
| 89, HCl | H | 3-(phénylsulfonyl)phényle | H | MS : 419,2 (ES+) |
| 90, HCl | H | 2-trifluorométhoxyphényle | H | MS : 363,1 (ES+) |

| | | | | |
|---|---|---|---|---|
| 91, HCl | H | 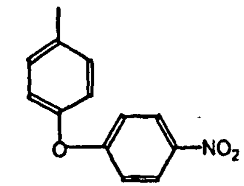 | H | MS : 416,2 (ES+) |
| 92, HCl | H | 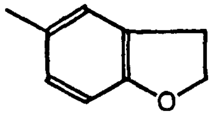 | H | MS : 321,2 (ES+) |
| 93, HCl | H | 4-(hydroxyéthoxy)phényle | H | MS : 339,1 (ES+) |
| 94, HCl | H | 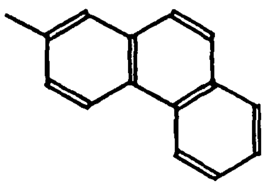 | H | MS : 379,2 (ES+) |
| 95, HCl | H | 3-nitro-4-phénylthio-phényle | H | MS : 432,1 (ES+) |
| 96, HCl | H | $R_6 + R_7$ = 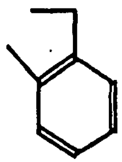 l'atome de carbone $sp_3$ étant relié à CS | | MS : 291,1 (ES+) |

| | | | | |
|---|---|---|---|---|
| 97, HCl | H | 4-(difluorométhoxy)phényle | H | F = 278,5 - 279 °C RMN 300 MHz (CDCl3) : 3,1 - 3,3 (2 H, m) ; 4,1 - 4,3 (2 H, m) ; 6,7 (1 H, t, J = 72,6 Hz) ; 7,1 - 7,6 (8 H, m) ; 8,1 (1 H, d, J = 8,7 Hz) ; 14,7 (1 H, s) MS : 345,1 (ES+) |
| 98, HCl | H | 3-méthyl-benzothièn-2-yle | H | MS : 349,1 (ES+) |
| 99, HCl | H | 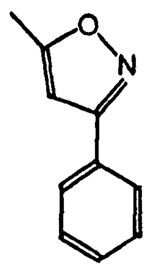 | H | MS : 346,1 (ES+) |
| 100 | H | 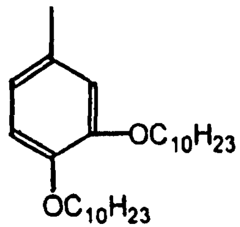 | H | MS : 591,4 (ES+) |
| 101, HCl | H | 4-(phénylsulfonyl)phényle | H | MS : 419,1 (ES+) |

| | | | | |
|---|---|---|---|---|
| 102 | H | 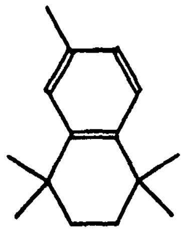 | H | MS : 389,2 (ES+) |
| 103, HCl | H | 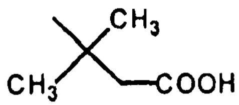 | H | MS : 303,1 (ES+) |
| 104 | H | 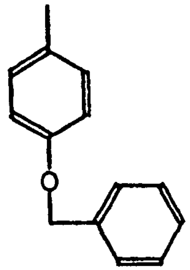 | H | MS : 383,4 (ES-) |
| 105 | H | 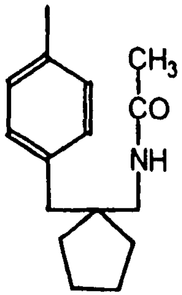 | H | MS : 432,5 (ES+) |

| | | | | |
|---|---|---|---|---|
| 106 | H | 4-(diéthylamino)phényle | H | MS : 350,4 (ES+) |
| 107 | H | phényle | 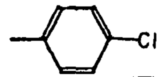 | MS : 389 (ES+) |
| 108 | H | 4-morpholinophényle | H | MS : 364,4 (ES+) |
| 109 | H | 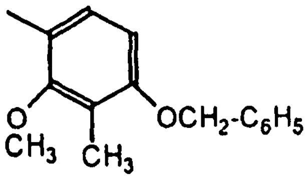 | H | MS : 429,3 (ES+) |
| 110 | H | 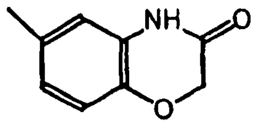 | H | MS : 350,3 (ES+), 348,3 (ES-) |
| 111 | H | 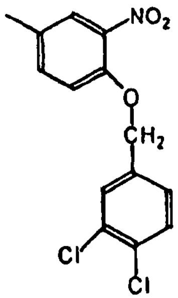 | H | MS : 498,1 et 500,1 (ES+) |

| | | | | |
|---|---|---|---|---|
| 112 | H | 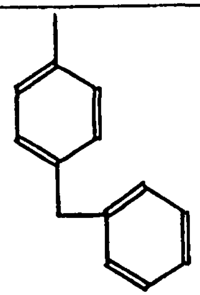 | H | MS : 369,4 (ES+) |
| 113 | H | 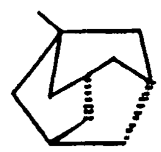 | H | MS : 337,4 (ES+), 335,4 (ES-) |
| 114 | H | 4-pyrrolidinophényle | H | MS : 348,2 (ES+) |
| 115 | H | 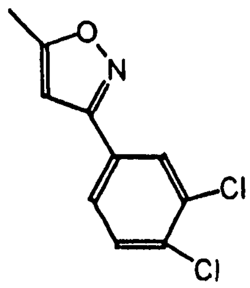 | H | MS : 432,1 (ES+) (+$H_2O$) |
| 116 | H | 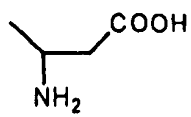 | H | MS : 290,2 (ES+), 288,3 (ES-) |

| | | | | |
|---|---|---|---|---|
| 117 | H | 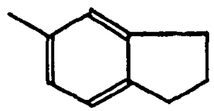 | H | MS : 319,2 (ES+), 317,3 (ES-) |
| 118, HCl | H | 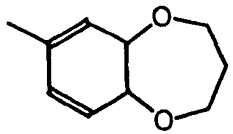 | H | MS : 351,1 (ES+) |
| 119 | H | 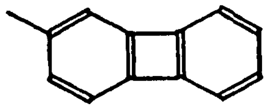 | H | MS : 353,3 (ES+) |

**Revendications**

**1.** Dérivé de benzodiazépine de formule I :

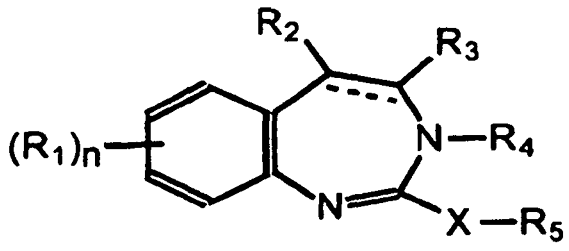

I

dans laquelle
les pointillés indiquent la présence éventuelle d'une double liaison ;
$R_1$ représente ($C_1$-$C_{18}$)alkyle éventuellement halogéné, ($C_1$-$C_{10}$)alcoxy éventuellement halogéné, halogène, nitro, hydroxy ou ($C_6$-$C_{18}$)aryle (éventuellement substitué par ($C_1$-$C_{10}$)alkyle éventuellement halogéné, ($C_1$-$C_{12}$)alcoxy éventuellement halogéné, halogène, nitro ou hydroxy) ;
n représente 0, 1, 2, 3 ou 4 ;
$R_2$ et $R_3$ représentent indépendamment l'un de l'autre hydrogène ; ($C_1$-$C_{18}$)alkyle éventuellement halogéné ; ($C_1$-$C_{18}$)alcoxy ; ($C_6$-$C_{18}$)aryle ; ($C_6$-$C_{18}$)aryl-($C_1$-$C_{12}$)alkyle ; hétéroaryle ; hétéroaryl($C_1$-$C_{12}$)alkyle ; ($C_6$-$C_{18}$) aryloxy ; ($C_6$-$C_{18}$)aryl-($C_1$-$C_{12}$)alcoxy ; hétéroaryloxy ; ou hétéroaryl($C_1$-$C_{12}$)alcoxy ; dans lesquels les parties aryle et hétéroaryle de ces radicaux sont éventuellement substituées par halogène, ($C_1$-$C_{12}$)alcoxy éventuellement halogéné, ($C_1$-$C_{12}$)alkyle éventuellement halogéné, nitro et hydroxy ;
X représente S, O ou -NT où T représente un atome d'hydrogène, ($C_1$-$C_{12}$)alkyle, ($C_6$-$C_{18}$)aryle, ($C_6$-$C_{18}$) aryl-($C_1$-$C_{12}$)alkyle ou ($C_6$-$C_{18}$)arylcarbonyle ;
$R_4$ et $R_5$ forment ensemble le groupe -$CR_6$=$CR_7$- dans lequel $CR_6$ est relié à X et dans lequel:

$R_6$ représente un atome d'hydrogène ; ($C_1$-$C_{18}$)alkyle ; ($C_3$-$C_{12}$)cycloalkyle ; ($C_6$-$C_{18}$)aryle ; carboxy-($C_1$-$C_{12}$) alkyle ; ($C_1$-$C_{12}$)alcoxy-carbonyl-($C_1$-$C_{12}$)alkyle ; hétéroaryle ; ($C_6$-$C_{18}$)aryl-($C_1$-$C_{12}$)alkyle ; et hétéroaryl-($C_1$-$C_{12}$)alkyle ; dans lesquels les parties aryle et hétéroaryle de ces radicaux sont éventuellement substituées par ($C_1$-$C_{12}$)alkyle, ($C_1$-$C_{12}$)alcoxy, hydroxy, nitro, halogène ou di($C_1$-$C_{12}$)alcoxyphosphoryl($C_1$-$C_{12}$) alkyle ;
$R_7$ représente un atome d'hydrogène ; hydroxy ; di($C_1$-$C_{12}$)alkylamino($C_1$-$C_{12}$)alkyle ; ($C_1$-$C_{18}$)alkyle éventuellement halogéné ; carboxy ; carboxy($C_1$-$C_{12}$)alkyle éventuellement substitué par amino ; ($C_1$-$C_{12}$) alcoxycarbonyle ; ($C_6$-$C_{18}$)aryle ; hétéroaryle ; ($C_6$-$C_{18}$)aryl-($C_1$-$C_{12}$)alkyle ; ou hétéroaryl-($C_1$-$C_{12}$)alkyle ; ($C_6$-$C_{18}$)aryle condensé à un hétérocycle insaturé, éventuellement substitué sur la partie hétérocycle par oxo ; ($C_3$-$C_{12}$)cycloalkyle ;

dans lesquels les parties aryle, hétérocycle, cycloalkyle et hétéroaryle de ces radicaux sont éventuellement substituées par halogène ; hydroxy ; hydroxy-($C_1$-$C_{12}$)alcoxy ; ($C_1$-$C_{12}$)alkyle éventuellement halogéné ; ($C_1$-$C_{12}$)alcoxy éventuellement halogéné ; carboxy ; ($C_1$-$C_{12}$)alcoxycarbonyle ; nitro ; cyano ; cyano($C_1$-$C_{18}$)alkyle ; ($C_1$-$C_{18}$) alkylcarbonyloxy ; ($C_2$-$C_{12}$)alkylène ; ($C_1$-$C_{12}$)alkylènedioxy ; ($C_1$-$C_{12}$)alkylthio ; ($C_6$-$C_{18}$)arylthio éventuellement substitué par un ou plusieurs substituants Su tels que définis ci-dessus ; di($C_1$-$C_{12}$)alkylamino ; un groupe de formule :

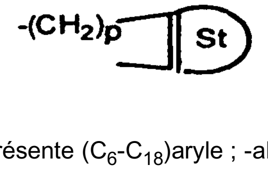

dans lequel p = 0, 1, 2, 3 ou 4 et où St représente ($C_6$-$C_{18}$)aryle ; -alk-Cy-NH-$SO_2$-Ar où alk représente ($C_1$-$C_{12}$) alkyle. Cy représente ($C_3$-$C_{12}$)cycloalkyle éventuellement substitué par un ou plusieurs substituants Su tels que définis ci-dessous et Ar représente ($C_6$-$C_{18}$)aryle éventuellement substitué par un ou plusieurs substituants Su tels que définis ci-dessous ; -Cy-alk-NH-$SO_2$-Ar où Cy, alk et Ar sont tels que définis ci-dessus ; -alk-Cy où alk et Cy sont tels que définis ci-dessus ; -alk-Cy-alk'-NH-CO-alk" où alk et Cy sont tels que définis ci-dessus et alk', alk"

représentent indépendamment ($C_1$-$C_{12}$)alkyle ; di($C_1$-$C_{12}$)alcoxyphosphoryl($C_1$-$C_{12}$)alkyle ; ($C_6$-$C_{18}$)aryle éventuellement substitué par un ou plusieurs substituants Su tels que définis ci-dessous ; ($C_6$-$C_{18}$)aryloxy éventuellement substitué par un ou plusieurs substituants Su tels que définis ci-dessous ; ($C_6$-$C_{18}$)arylcarbonyle éventuellement substitué par un ou plusieurs substituants Su tels que définis ci-dessous ; ($C_6$-$C_{18}$)arylsulfonyle éventuellement substitué par un ou plusieurs substituants Su tels que définis ci-dessous ; ($C_6$-$C_{18}$)aryl-($C_1$-$C_{12}$)alcoxy dont la partie aryle est éventuellement substituée par un ou plusieurs substituants Su tels que définis ci-dessous ; hétérocycle saturé éventuellement substitué par un ou plusieurs substituants Su tels que définis ci-dessous ; ($C_6$-$C_{18}$) aryl-($C_1$-$C_{12}$)alkyle éventuellement substitué par un ou plusieurs substituants Su tels que définis ci-dessous ;
Su est choisi parmi hydroxy, halogène, cyano, nitro, ($C_1$-$C_{12}$)alkyle éventuellement halogéné et ($C_1$-$C_{12}$)alcoxy éventuellement halogéné ;
ou bien $R_6$ et $R_7$ forment ensemble une chaîne alkylène en $C_3$-$C_{12}$ éventuellement interrompue par un atome d'azote lequel est éventuellement substitué par ($C_1$-$C_{12}$)alkyle ou ($C_6$-$C_{18}$)aryle ou -($C_6$-$C_{18}$)aryl-($C_1$-$C_{12}$)alkyle, le cycle formé par $CR_6$=$CR_7$ étant éventuellement condensé à ($C_6$-$C_{18}$)aryle (les parties aryle de ces radicaux étant éventuellement substituées par halogène, nitro, hydroxy, ($C_1$-$C_{12}$)alkyle: éventuellement halogéné ou ($C_1$-$C_{12}$)alcoxy éventuellement halogéné) ;
et leurs sels pharmaceutiquement acceptables avec des acides ou des bases, étant entendu que les composés suivants sont exclus du cadre de l'invention:

(a) X = S ; n = 0 ; $R_2$ représente méthyle et $R_3$ représente un atome d'hydrogène ; $R_4$ et $R_5$ forment ensemble le groupe -$CR_6$=$CR_7$- dans lequel $CR_6$ est relié à X, $R_6$ et $R_7$ forment ensemble une chaîne $-(CH_2)_3-$ ou $-(CH_2)_4-$ ou bien $R_6$ représente un atome d'hydrogène ou un groupe propyle et $R_7$ est un groupe phényle éventuellement substitué par $-OCH_3$ ou un groupe hydroxy.

2. Composé selon la revendication 1, **caractérisé en ce que** X représente -NT où T est tel que défini à la revendication 1 et $R_4$ et $R_5$ forment ensemble -$CR_6$=$CR_7$.

3. Composé selon la revendication 1 ou la revendication 2, **caractérisé en ce que** $R_3$ représente un atome d'hydrogène.

4. Composé selon l'une quelconque des revendications 1 à 3, **caractérisé en ce que** $R_2$ représente un atome d'hydrogène ou un groupe ($C_6$-$C_{10}$)aryle éventuellement substitué par halogène, ($C_1$-$C_6$)alcoxy, ($C_1$-$C_6$)alkyle éventuellement halogéné, nitro et hydroxy.

5. Composé selon l'une quelconque des revendications 1 à 4, **caractérisé en ce que** n est 0 ou 1 et $R_1$ représente un atome d'halogène.

6. Composé selon l'une quelconque des revendications 1 et 3 à 5, **caractérisé en ce que** X représente S :
$R_4$ et $R_5$ forment ensemble le groupe -$CR_6$=$CR_7$- dans lequel
$R_6$ représente un atome d'hydrogène, ($C_1$-$C_6$)alkyle, ($C_6$-$C_{10}$)aryle (éventuellement substitué par halogène, hydroxy, nitro, ($C_1$-$C_6$)alkyle ou ($C_1$-$C_6$)alcoxy), carboxy-($C_1$-$C_6$)alkyle, ou bien ($C_1$-$C_6$)alcoxycarbonyl-($C_1$-$C_6$)alkyle ; et
$R_7$ représente un atome d'hydrogène ; hydroxy ; di($C_1$-$C_6$)alkylamino($C_1$-$C_6$)alkyle ; ($C_1$-$C_{10}$)alkyle ; ($C_1$-$C_6$) alcoxycarbonyle ; ($C_6$-$C_{10}$)aryle ; hétéroaryle ; ($C_6$-$C_{10}$)aryl-($C_1$-$C_6$)alkyle ; les parties aryle et hétéroaryle de ces radicaux étant éventuellement substituées par ($C_1$-$C_6$)alcoxycarbonyle, halogène, hydroxy, ($C_1$-$C_6$)alkyle, ($C_6$-$C_{10}$) aryle, (ce dernier étant éventuellement substitué par halogène, ($C_1$-$C_6$)alkyle éventuellement halogéné, ($C_1$-$C_6$) alcoxy ou nitro) ou ($C_6$-$C_{10}$)aryle condensé à un hétérocycle aromatique ou insaturé de 5 à 7 chaînons comprenant un, deux ou trois hétéroatomes endocycliques choisis parmi O, N et S ; ou bien $R_6$ et $R_7$ forment ensemble une chaîne alkylène interrompue par un atome d'azote éventuellement substitué par ($C_6$-$C_{10}$)aryl-($C_1$-$C_6$)alkyle dans lequel la partie aryle est éventuellement substituée par halogène, ($C_1$-$C_6$)alkyle éventuellement halogéné, ($C_1$-$C_6$) alcoxy, hydroxy ou nitro.

7. Composé selon l'une quelconque des revendications 1 à 5, **caractérisé en ce que** X représente -NT ; et $R_4$ et $R_5$ forment ensemble le groupe -$CR_6$=$CR_7$-dans lequel $R_6$ représente un atome d'hydrogène et $R_7$ représente hydroxyle ou ($C_6$-$C_{10}$)aryle éventuellement substitué par halogène, nitro, hydroxy, ($C_1$-$C_6$)alkyle éventuellement halogéné ou ($C_1$-$C_6$)alcoxy.

8. Composé selon la revendication 1, choisi parmi les :

3-(biphényl-4-yl)-5,6-dihydrothiazolo[2,3-b]-1.3-benzodiazépine:
3-(2-furyl)-5,6-dihydrothiazolo[2,3-b]-1,3-benzodiazépine ;

3-[4-(éthoxy-carbonyl)phényl]-5,6-dihydrothiazolo-[2,3-b]-1,3-benzodiazépine ;
3-(biphényl-3-yl)-5,6-dihydrothiazolo[2,3-b]-1,3-benzodiazépine ;
3-(3,4-dihydroxyphényl)-5,6-dihydrothiazolo[2,3-b]-1,3-benzodiazépine ; et
3-(biphényl-4-yl)-7-chloro-5,6-dihydrothiazolo[2,3-b]-1,3-benzodiazépine.

**9.** Procédé pour la préparation de composés de formule I selon la revendication 1, dans laquelle X représente S et $R_4$ et $R_5$ forment ensemble le groupe $-CR_6=CR_7-$, comprenant la réaction d'une thione de formule IIa :

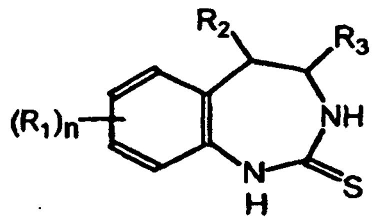

IIa

dans laquelle n, $R_1$, $R_2$ et $R_3$ sont tels que définis à la revendication 1 avec une α-halogénocétone de formule IVb :

$R_7$-CO-CH$R_6$-Hal$^3$ IVb

dans laquelle $R_6$ et $R_7$ sont tels que définis à la revendication 1, et Hal$^3$ représente un atome d'halogène, dans un acide carboxylique aliphatique en $C_2$-$C_6$ à une température comprise entre 90 et 130° C.

**10.** Procédé selon la revendication 11, **caractérisé en ce que** l'acide carboxylique aliphatique est l'acide acétique.

**11.** Procédé selon l'une quelconque des revendications 9 à 10, **caractérisé en ce que** la température est maintenue entre 100 et 125° C.

**12.** Procédé pour la préparation de composés de formule 1 selon la revendication 1 dans laquelle X représente -NH, $R_4$ et $R_5$ forment ensemble le groupe $-CR_6=CR_7-$, et $R_7$ n'est pas hydroxy, comprenant la réaction d'un sulfure de formule V :

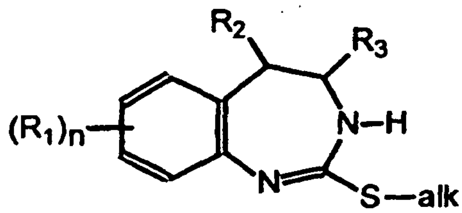

V

dans laquelle n, $R_1$, $R_2$ et $R_3$ sont tels que définis à la revendication 1, $R_4$ et $R_5$ forment ensemble le groupe $-CR_6=CR_7-$, et alk représente ($C_1$-$C_6$)alkyle, avec un dérivé protégé de la cétone de formule VI :

$NH_2$-CH$R_6$-CO-$R_7$ VI

dans lequel le groupe carbonyle est protégé par un groupement protecteur labile en milieu acide, $R_6$ et $R_7$ étant tels que définis à la revendication 1, puis le traitement par un acide du composé résultant.

**13.** Procédé pour la préparation de composés de formule I selon la revendication 1 dans laquelle X représente -NT où T n'est pas un atome d'hydrogène, $R_4$ et $R_5$ forment ensemble le groupe $-CR_6=CR_7$, et $R_7$ représente hydroxy, comprenant la réaction d'un sulfure de formule V :

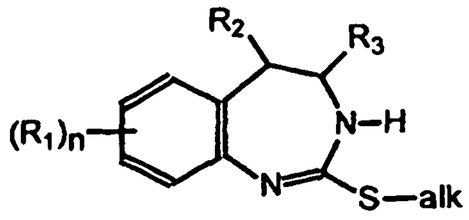

V

dans laquelle n, $R_1$, $R_2$ et $R_3$ sont tels que définis à la revendication 1, et alk représente $(C_1-C_6)$alkyle, avec un dérivé de formule VIII :

$HTN-CHR_6-CO-Y$ VIII

dans laquelle T et $R_6$ sont tels que définis à la revendication 1 et Y est un groupe partant, à une température comprise entre 50 et 150° C, de préférence à une température comprise entre 60 et 100° C.

**14.** Procédé selon la revendication 12 comprenant en outre la réaction du composé obtenu par mise en oeuvre du procédé de la revendication 12, avec un réactif halogéné de formule Hal-T où T représente $(C_1-C_6)$alkyle. $(C_6-C_{10})$ aryle ou $(C_6-C_{10})$aryl-$(C_1-C_6)$alkyle et Hal est un atome d'halogène, en présence d'une base, de façon à synthétiser le composé correspondant de formule 1 dans lequel T représente $(C_1-C_6)$alkyle, $(C_6-C_{10})$aryle ou $(C_6-C_{10})$ aryl-$(C_1-C_6)$alkyle.

**15.** Composition pharmaceutique contenant une quantité efficace d'au moins un composé de formule (I) selon l'une quelconque des revendications 1 à 8 en association avec au moins un véhicule pharmaceutiquement acceptable.

**16.** Utilisation d'un composé de formule I selon l'une quelconque des revendications 1 à 8 pour la préparation d'un médicament destiné à prévenir ou traiter les dyslipidémies, fathérosclérose, le diabète et ses complications.

**Patentansprüche**

**1.** Benzodiazepin-Derivat der Formel I:

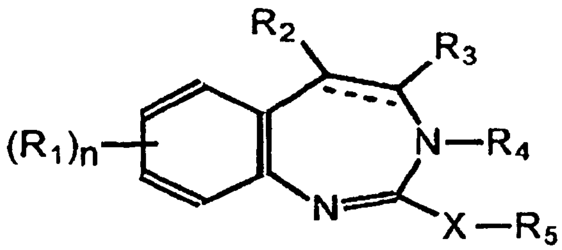

I

worin
die gestrichelte Linie das optionale Vorliegen einer Doppelbindung angibt,
$R_1$ gegebenenfalls halogeniertes $(C_1-C_{18})$Alkyl, gegebenenfalls halogeniertes $(C_1-C_{18})$Alkoxy, Halogen, Nitro, Hydroxy oder $(C_6-C_{18})$Aryl (das gegebenenfalls mit gegebenenfalls halogeniertem $(C_1-C_{10})$Alkyl, gegebenenfalls halogeniertem $(C_1-C_{12})$Alkoxy, Halogen, Nitro oder Hydroxy substituiert ist) bedeutet;
n 0, 1, 2, 3 oder 4 bedeutet;
$R_2$ und $R_3$ unabhängig voneinander Wasserstoff; gegebenenfalls halogeniertes $(C_1-C_{18})$Alkyl; $(C_1-C_{18})$Alkoxy; $(C_6-C_{18})$Aryl; $(C_6-C_{18})$Aryl-$(C_1-C_{12})$alkyl; Heteroaryl; Heteroaryl$(C_1-C_{12})$alkyl; $(C_6-C_{18})$Aryloxy; $(C_6-C_{18})$-Aryl $(C_1-C_{12})$alkoxy; Heteroaryloxy; oder Heteroaryl$(C_1-C_{12})$alkoxy bedeuten; wobei die Aryl- und Heteroarylteile dieser Reste gegebenenfalls mit Halogen, gegebenenfalls halogeniertem $(C_1-C_{12})$Alkoxy, gegebenenfalls halogeniertem $(C_1-C_{12})$Alkyl, Nitro und Hydroxy substituiert sind;
X S, O oder -NT bedeutet, wobei T ein Wasserstoffatom, $(C_1-C_{12})$-Alkyl, $(C_6-C_{18})$Aryl, $(C_6-C_{18})$Aryl$(C_1-C_{12})$alkyl

oder $(C_6-C_{18})$Arylcarbonyl bedeutet;
$R_4$ und $R_5$ zusammen die Gruppe $-CR_6=CR_7-$ bilden, worin $CR_6$ an X gebunden ist und worin:

$R_6$ ein Wasserstoffatom; $(C_1-C_{18})$Alkyl; $(C_3-C_{12})$Cycloalkyl; $(C_6-C_{18})$-Aryl; Carboxy$(C_1-C_{12})$alkyl; $(C_1-C_{12})$Alkoxycarbonyl$(C_1-C_{12})$alkyl; Heteroaryl; $(C_6-C_{18})$Aryl$(C_1-C_{12})$alkyl; und Heteroaryl$(C_1-C_{12})$aryl bedeutet; wobei die Aryl- und Heteroarylteile dieser Reste gegebenenfalls mit $(C_1-C_{12})$Alkyl, $(C_1-C_{12})$Alkoxy, Hydroxy, Nitro, Halogen oder Di$(C_1-C_{12})$alkoxyphosphoryl-$(C_1-C_{12})$alkyl substituiert sind;
$R_7$ ein Wasserstoffatom; Hydroxy; Di$(C_1-C_{12})$alkylamino$(C_1-C_{12})$alkyl; gegebenenfalls halogeniertes $(C_1-C_{18})$ Alkyl; Carboxy; gegebenenfalls mit Amino substituiertes Carboxy$(C_1-C_{12})$alkyl; $(C_1-C_{12})$Alkoxycarbonyl; $(C_6-C_{18})$Aryl; Heteroaryl; $(C_6-C_{18})$Aryl$(C_1-C_{12})$alkyl; oder Heteroaryl$(C_1-C_{12})$-alkyl; an ein ungesättigtes Heterocyclus kondensiertes $(C_6-C_{18})$Aryl, das gegebenenfalls am Heterocyclusteil mit Oxo substituiert ist; $(C_3-C_{12})$ Cycloalkyl bedeutet;

worin die Aryl-, Heterocyclus-, Cycloalkyl- und Heteroarylteile dieser Reste gegebenenfalls mit Halogen; Hydroxy; Hydroxy$(C_1-C_{12})$alkoxy; gegebenenfalls halogeniertem $(C_1-C_{12})$Alkyl; gegebenenfalls halogeniertem $(C_1-C_{12})$Alkoxy; Carboxy; $(C_1-C_{12})$Alkoxycarbonyl; Nitro; Cyano; Cyano$(C_{1-}C_{18})$alkyl; $(C_1-C_{18})$Alkylcarbonyloxy; $(C_2-C_{12})$Alkylen; $(C_1-C_{12})$Alkylendioxy; $(C_1-C_{12})$Alkylthio; gegebenenfalls mit einem oder mehreren Substituenten Su, wie nachstehend definiert, substituiertes $(C_6-C_{18})$Arylthio; Di$(C_1-C_{12})$-alkylamino; einer Gruppe der Formel:

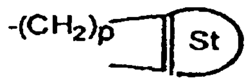

worin p = 1, 2, 3 oder 4 und wobei St $(C_6-C_{18})$Aryl bedeutet; -alk-Cy-NH-$SO_2$-Ar, wobei alk $(C_1-C_{12})$Alkyl bedeutet, Cy $(C_3-C_{12})$Cycloalkyl, das gegebenenfalls mit einem oder mehreren Substituenten Su, wie nachstehend definiert, substituiert ist, bedeutet und Ar gegebenenfalls mit einem oder mehreren Substituenten Su, wie nachstehend definiert, substituiertes $(C_6-C_{18})$Aryl bedeutet; Cy-alk-NH-$SO_2$-Ar, wobei alk und Ar wie vorstehend definiert sind; -alk-Cy, wobei alk und Cy wie vorstehend definiert sind; -alk-Cy-alk'-NH-CO-alk", wobei alk und Cy wie vorstehend definiert sind und alk', alk" unabhängig $(C_1-C_{12})$Alkyl bedeuten; Di$(C_1-C_{12})$-alkoxyphosphoryl$(C_1-C_{12})$alkyl; gegebenenfalls mit einem oder mehreren Substituenten Su, wie nachstehend definiert, substituiertes $(C_6-C_{18})$Aryl; gegebenenfalls mit einem oder mehreren Substituenten Su, wie nachstehend definiert, substituiertes $(C_6-C_{18})$Aryloxy; gegebenenfalls mit einem oder mehreren Substituenten Su, wie nachstehend definiert, substituiertes $(C_6-C_{18})$Arylcarbonyl; gegebenenfalls mit einem oder mehreren Substituenten Su, wie nachstehend definiert, substituiertes $(C_6-C_{18})$Arylsulfonyl; $(C_6-C_{18})$Aryl$(C_1-C_{12})$alkoxy, dessen Arylteil gegebenenfalls mit einem oder mehreren Substituenten Su, wie nachstehend definiert, substituiert ist; einen gesättigten Heterocyclus, der gegebenenfalls mit einem oder mehreren Substituenten Su, wie nachstehend definiert, substituiert ist; gegebenenfalls mit einem oder mehreren Substituenten Su, wie nachstehend definiert, substituiertes $(C_6-C_{18})$Aryl$(C_1-C_{12})$alkyl; substituiert sind;
Su aus Hydroxy, Halogen, Cyano, Nitro, gegebenenfalls halogeniertem $(C_1-C_{12})$Alkyl und gegebenenfalls halogeniertem $(C_1-C_{12})$Alkoxy ausgewählt ist,
oder auch $R_6$ und $R_7$ zusammen eine $C_3$-$C_{12}$-Alkylen-Kette bilden, die gegebenenfalls durch ein gegebenenfalls mit $(C_1-C_{12})$Alkyl oder $(C_6-C_{18})$Aryl oder $(C_6-C_{18})$Aryl$(C_1-C_{12})$alkyl substituiertes Stickstoffatom unterbrochen ist, wobei der durch $CR_6=CR_7$ gebildete Ring gegebenenfalls an $(C_6-C_{18})$Aryl kondensiert ist (wobei die Arylteile dieser Reste gegebenenfalls mit Halogen, Nitro, Hydroxy, gegebenenfalls halogeniertem $(C_1-C_{12})$-Alkyl oder gegebenenfalls halogeniertem $(C_1-C_{12})$Alkoxy substituiert sind),
und ihre pharmazeutisch unbedenklichen Salze mit Säuren oder Basen,
wobei die folgenden Verbindungen selbstverständlich vom Umfang der Erfindung ausgeschlossen sind:

(a) X = S, n = 0, $R_2$ Methyl bedeutet, und $R_3$ ein Wasserstoffatom bedeutet, $R_4$ und $R_5$ zusammen die Gruppe $-CR_6=CR_7-$ bilden, worin $CR_6$ an X gebunden ist, $R_6$ und $R_7$ zusammen eine Kette $-(CH_2)_3-$ oder $-(CH_2)_4-$ bilden oder $R_6$ ein Wasserstoffatom oder eine Propylgruppe bedeutet und $R_7$ eine gegebenenfalls mit $-OCH_3$ oder einer Hydroxygruppe substituierte Phenylgruppe ist.

**2.** Verbindung nach Anspruch 1, **dadurch gekennzeichnet, dass** X -NT bedeutet, worin T wie im Anspruch 1 definiert ist, und $R_4$ und $R_5$ zusammen $-CR_6=CR_7$ bilden.

**3.** Verbindung nach Anspruch 1 oder Anspruch 2, **dadurch gekennzeichnet, dass** $R_3$ ein Wasserstoffatom bedeutet.

4. Verbindung nach einem der Ansprüche 1 bis 3, **dadurch gekennzeichnet, dass** $R_2$ ein Wasserstoffatom, eine gegebenenfalls mit Halogen substituierte $(C_6$-$C_{10})$Aryl-, $(C_1$-$C_6)$Alkoxy-, gegebenenfalls halogeniertes $(C_1$-$C_6)$Alkyl-, Nitro- und Hydroxy-Gruppe bedeutet.

5. Verbindung nach einem der Ansprüche 1 bis 4, **dadurch gekennzeichnet, dass** n 0 oder 1 ist und $R_1$ ein Halogenatom bedeutet.

6. Verbindung nach einem der Ansprüche 1 und 3 bis 5, **dadurch gekennzeichnet, dass** X S bedeutet;
$R_4$ und $R_5$ zusammen die Gruppe $-CR_6=CR_7$ bilden, worin
$R_6$ ein Wasserstoffatom, $(C_1$-$C_6)$Alkyl, $(C_6$-$C_{10})$Aryl (das gegebenenfalls mit Halogen, Hydroxy, Nitro, $(C_1$-$C_6)$Alkyl oder $(C_1$-$C_6)$Alkoxy substituiert ist), Carboxy$(C_1$-$C_6)$alkyl oder $(C_1$-$C_6)$Alkoxycarbonyl$(C_1$-$C_6)$alkyl bedeutet; und
$R_7$ ein Wasserstoffatom; Hydroxy; Di$(C_1$-$C_6)$alkylamino$(C_1$-$C_6)$alkyl; $(C_1$-$C_{10})$Alkyl; $(C_1$-$C_6)$Alkoxycarbonyl; $(C_6$-$C_{10})$Aryl; Heteroaryl; $(C_6$-$C_{10})$Aryl-$(C_1$-$C_6)$alkyl bedeutet, wobei die Aryl- und Heteroarylteile dieser Reste gegebenenfalls mit $(C_1$-$C_6)$Alkoxycarbonyl, Halogen, Hydroxy, $(C_1$-$C_6)$Alkyl, $(C_6$-$C_{10})$Aryl (das gegebenenfalls mit Halogen, gegebenenfalls halogeniertem $(C_1$-$C_6)$Alkyl, $(C_1$-$C_6)$Alkoxy oder Nitro substituiert ist) oder $(C_6$-$C_{10})$-Aryl, das an ein aromatisches oder ungesättigtes Heterocyclus mit 5 bis 7 Ringgliedern, der ein, zwei oder drei aus O, N und S ausgewählte endocyclische Heteroatome enthält, kondensiert ist, substituiert sind, oder $R_6$ und $R_7$ zusammen auch eine Alkylen-Kette bilden, die durch ein gegebenenfalls mit $(C_6$-$C_{10})$Aryl$(C_1$-$C_6)$alkyl substituiertes Stickstoffatom unterbrochen ist, wobei der Arylteil gegebenenfalls mit Halogen, gegebenenfalls halogeniertem $(C_1$-$C_6)$Alkyl, gegebenenfalls halogeniertem $(C_1$-$C_6)$Alkoxy, Hydroxy oder Nitro substituiert ist.

7. Verbindung nach einem der Ansprüche 1 bis 5, **dadurch gekennzeichnet, dass** X -NT bedeutet und $R_4$ und $R_5$ zusammen die Gruppe $-CR_6=CR_7-$ bilden, worin $R_6$ ein Wasserstoffatom bedeutet und $R_7$ Hydroxy oder gegebenenfalls mit Halogen, Nitro, Hydroxy substituiertes $(C_6$-$C_{10})$-Aryl, gegebenenfalls halogeniertes $(C_1$-$C_6)$Alkyl oder $(C_1$-$C_6)$Alkoxy bedeutet.

8. Verbindung nach Anspruch 1, die aus:

   3-(Biphenyl-4-yl)-5,6-dihydrothiazolo[2,3-b]-1,3-benzodiazepin;
   3-(2-Furyl)-5,6-dihydrothiazolo[2,3-b]-1,3-benzodiazepin;
   3-[4-(Ethoxycarbonyl)phenyl]-5,6-dihydrothiazolo[2,3-b]-1,3-benzodiazepin;
   3-(Biphenyl-3-yl)-5,6-dihydrothiazolo[2,3-b]-1,3-benzodiazepin;
   3-(3,4-Dihydroxyphenyl)-5,6-dihydrothiazolo[2,3-b]-1,3-benzodiazepin; und
   3-(Biphenyl-4-yl)-7-chlor-5,6-dihydrothiazolo[2,3-b]-1,3-benzodiazepin,

   ausgewählt ist

9. Verfahren zur Herstellung von Verbindungen der Formel I nach Anspruch 1, worin X S bedeutet und $R_4$ und $R_5$ zusammen die Gruppe $-CR_6=CR_7-$ bilden, umfassend die Umsetzung eines Thions der Formel IIa:

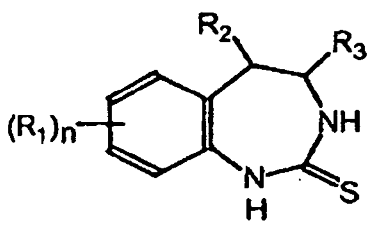

IIa

worin n, $R_1$, $R_2$ und $R_3$ wie im Anspruch 1 definiert sind, mit einem $\alpha$-Halogenketon der Formel IVb:

$R_7$-CO-CH$R_6$-Hal$^3$ IVb

worin $R_6$ und $R_7$ wie im Anspruch 1 definiert sind und Hal$^3$ ein Halogenatom bedeutet, in einer aliphatischen $C_2$-$C_6$-Carbonsäure bei einer Temperatur zwischen 90 und 130°C.

10. Verfahren nach Anspruch 11, **dadurch gekennzeichnet, dass** es sich bei der aliphatischen Carbonsäure um

Essigsäure handelt.

**11.** Verfahren nach einem der Ansprüche 9 bis 10, **dadurch gekennzeichnet, dass** die Temperatur zwischen 100 und 125°C gehalten wird.

**12.** Verfahren zur Herstellung von Verbindungen der Formel I nach Anspruch 1, worin X -NH bedeutet, $R_4$ und $R_5$ zusammen die Gruppe $-CR_6=CR_7-$ bilden und $R_7$ nicht Hydroxy ist, umfassend die Umsetzung eines Sulfids der Formel V:

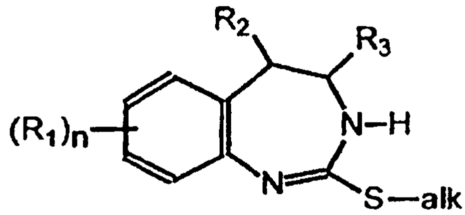

V

worin n, $R_1$, $R_2$ und $R_3$ wie im Anspruch 1 definiert sind, $R_4$ und $R_5$ zusammen die Gruppe $-CR_6=CR_7-$ bilden und alk $(C_1-C_6)$Alkyl bedeutet, mit einem geschützten Derivat des Ketons der Formel VI:

$NH_2-CHR_6-CO-R_7$ VI

worin die Carbonylgruppe durch eine in saurem Medium labile Schutzgruppe geschützt ist, $R_6$ und $R_7$ wie im Anspruch 1 definiert sind, und die anschließende Behandlung der so erhaltenen Verbindung mit einer Säure.

**13.** Verfahren zur Herstellung von Verbindungen der Formel I nach Anspruch 1, worin X -NT bedeutet, wobei T kein Wasserstoffatom ist, $R_4$ und $R_5$ zusammen die Gruppe $-CR_6=CR_7-$ bilden und $R_7$ Hydroxy bedeutet, umfassend die Umsetzung eines Sulfids der Formel V:

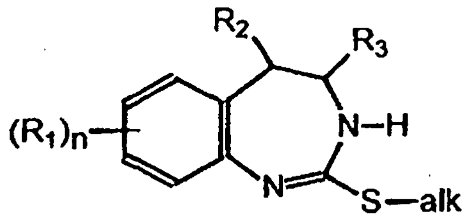

V

worin n, $R_1$, $R_2$ und $R_3$ wie im Anspruch 1 definiert sind und alk $(C_1-C_6)$Alkyl bedeutet, mit einem Derivat der Formel VIII:

$HTN-CHR_6-CO-Y$ VIII

worin T und $R_6$ wie im Anspruch 1 definiert sind und Y eine Abgangsgruppe ist, bei einer Temperatur zwischen 50 und 150°C, vorzugsweise bei einer Temperatur zwischen 60 und 100°C.

**14.** Verfahren nach Anspruch 12, umfassend ferner die Umsetzung der Verbindung, die mittels Durchführung des Verfahren des Anspruchs 12 erhalten wird, mit einem halogenierten Reagenz der Formel Hal-T, worin T $(C_1-C_6)$ Alkyl, $(C_6-C_{10})$Aryl oder $(C_6-C_{10})$Aryl$(C_1-C_6)$alkyl bedeutet und Hal ein Halogenatom ist, in Gegenwart einer Base, um die entsprechende Verbindung der Formel I zu synthetisieren, worin T $(C_1-C_6)$Alkyl, $(C_6-C_{10})$Aryl oder $(C_6-C_{10})$ Aryl$(C_1-C_6)$alkyl bedeutet.

**15.** Pharmazeutische Zusammensetzung, die eine wirksame Menge mindestens einer Verbindung der Formel (I) nach einem der Ansprüche 1 bis 8 in Verbindung mit mindestens einem pharmazeutisch unbedenklichen Träger enthält.

**16.** Verwendung einer Verbindung der Formel I nach einem der Ansprüche 1 bis 8 zur Herstellung eines Arzneimittels, das zur Vorbeugung oder Behandlung von Dyslipidämien, Atherosklerose, Diabetes und deren Komplikationen bestimmt ist.

## Claims

**1.** Benzodiazepine derivative of the formula I:

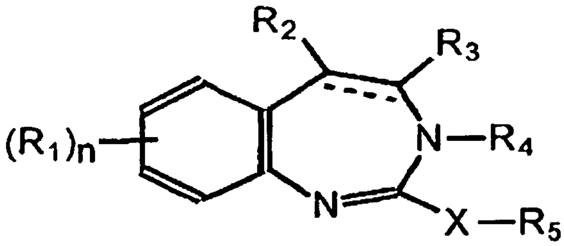

I

in which
the dashed lines indicate the possible presence of a double bond;
$R_1$ represents optionally halogenated ($C_1$-$C_{18}$)alkyl, optionally halogenated ($C_1$-$C_{18}$)alkoxy, halogen, nitro, hydroxyl or ($C_6$-$C_{18}$)aryl (optionally substituted by optionally halogenated ($C_1$-$C_{10}$)alkyl, optionally halogenated ($C_1$-$C_{12}$) alkoxy, halogen, nitro or hydroxyl);
n represents 0, 1, 2, 3 or 4;
$R_2$ and $R_3$ represent, independently of each other, hydrogen; optionally halogenated ($C_1$-$C_{18}$)alkyl; ($C_1$-$C_{18}$)alkoxy; ($C_6$-$C_{18}$)aryl; ($C_6$-$C_{18}$)aryl-($C_1$-$C_{12}$)alkyl; heteroaryl; heteroaryl($C_1$-$C_{12}$)alkyl; ($C_6$-$C_{18}$)aryloxy; ($C_6$-$C_{18}$)-aryl($C_1$-$C_{12}$) alkoxy; heteroaryloxy; or heteroaryl($C_1$-$C_{12}$)alkoxy; in which the aryl and heteroaryl portions of these radicals are optionally substituted by halogen, optionally halogenated ($C_1$-$C_{12}$)alkoxy, optionally halogenated ($C_1$-$C_{12}$)alkyl, nitro and hydroxyl;
X represents S, O or -NT in which T represents a hydrogen atom, ($C_1$-$C_{12}$)alkyl, ($C_6$-$C_{18}$)aryl, ($C_6$-$C_{18}$)aryl($C_1$-$C_{12}$) alkyl or ($C_6$-$C_{18}$)arylcarbonyl; $R_4$ and $R_5$ together form a group -$CR_6$=$CR_7$- in which $CR_6$ is linked to X and in which:

> $R_6$ represents a hydrogen atom; ($C_1$-$C_{18}$)alkyl; ($C_3$-$C_{12}$)cycloalkyl; ($C_6$-$C_{18}$)aryl; carboxy($C_1$-$C_{12}$)alkyl; ($C_1$-$C_{12}$) alkoxycarbonyl($C_1$-$C_{12}$)alkyl; heteroaryl; ($C_6$-$C_{18}$)aryl($C_1$-$C_{12}$)alkyl; and heteroaryl($C_1$-$C_{12}$)alkyl; in which the aryl and heteroaryl portions of these radicals are optionally substituted by ($C_1$-$C_{12}$)alkyl, ($C_1$-$C_{12}$)alkoxy, hydroxyl, nitro, halogen or di($C_1$-$C_{12}$)-alkoxyphosphoryl($C_1$-$C_{12}$)alkyl;
> $R_7$ represents a hydrogen atom; hydroxyl; di($C_1$-$C_{12}$)alkylamino-($C_1$-$C_{12}$)alkyl; optionally halogenated ($C_1$-$C_{18}$) alkyl; carboxyl; carboxy-($C_1$-$C_{12}$)alkyl optionally substituted by amino; ($C_1$-$C_{12}$)alkoxycarbonyl; ($C_6$-$C_{18}$)aryl; heteroaryl; ($C_6$-$C_{18}$)aryl($C_1$-$C_{12}$)alkyl; or heteroaryl($C_1$-$C_{12}$)-alkyl; ($C_6$-$C_{18}$)aryl fused to an unsaturated heterocycle, optionally substituted on the heterocycle portion with oxo; ($C_3$-$C_{12}$)cycloalkyl;

in which the aryl, heterocycle, cycloalkyl and heteroaryl portions of these radicals are optionally substituted by halogen; hydroxyl; hydroxy-($C_1$-$C_{12}$)alkoxy; optionally halogenated ($C_1$-$C_{12}$)alkyl; optionally halogenated ($C_1$-$C_{12}$) alkoxy; carboxyl; ($C_1$-$C_{12}$)alkoxycarbonyl; nitro; cyano; cyano-($C_1$-$C_{18}$)alkyl; ($C_1$-$C_{18}$)alkylcarbonyloxy; ($C_2$-$C_{12}$) alkylene; ($C_1$-$C_{12}$)alkylenedioxy; ($C_1$-$C_{12}$)alkylthio; ($C_6$-$C_{18}$)arylthio optionally substituted by one or more substituents Su as defined above; di($C_1$-$C_{12}$)alkylamino; a group of the formula:

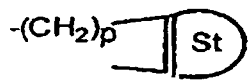

in which p = 0, 1, 2, 3 or 4 and in which St represents ($C_6$-$C_{18}$)aryl, -alk-Cy-NH-$SO_2$-Ar in which alk represents ($C_1$-$C_{12}$)alkyl, Cy represents ($C_3$-$C_{12}$)cycloalkyl optionally substituted by one or more substituents Su as defined below and Ar represents ($C_6$-$C_{18}$)aryl optionally substituted by one or more substituents Su as defined below; -Cy-alk-NH-$SO_2$-Ar in which Cy, alk and Ar are as defined above; -alk-Cy in which alk and Cy are as defined above;

-alk-Cy-alk'-NH-CO-alk" in which alk and Cy are as defined above and alk' and alk" represent, independently, $(C_1-C_{12})$alkyl; di$(C_1-C_{12})$alkoxyphosphoryl$(C_1-C_{12})$alkyl; $(C_6-C_{18})$aryl optionally substituted by one or more substituents Su as defined below; $(C_6-C_{18})$aryloxy optionally substituted by one or more substituents Su as defined below; $(C_6-C_{18})$arylcarbonyl optionally substituted by one or more substituents Su as defined below; $(C_6-C_{18})$arylsulfonyl optionally substituted by one or more substituents Su as defined below; $(C_6-C_{18})$aryl$(C_1-C_{12})$alkoxy in which the aryl portion is optionally substituted by one or more substituents Su as defined below; saturated heterocycle optionally substituted by one or more substituents Su as defined below; $(C_6-C_{18})$aryl$(C_1-C_{12})$alkyl optionally substituted by one or more substituents Su as defined below;
Su is chosen from hydroxyl, halogen, cyano, nitro, optionally halogenated $(C_1-C_{12})$alkyl and optionally halogenated $(C_1-C_{12})$alkoxy;
or alternatively $R_6$ and $R_7$ together form a $C_3-C_{12}$ alkylene chain optionally interrupted by a nitrogen atom which is optionally substituted by $(C_1-C_{12})$alkyl or $(C_6-C_{18})$aryl or $(C_6-C_{18})$aryl$(C_1-C_{12})$alkyl, the ring formed by $CR_6=CR_7$ being optionally fused to $(C_6-C_{18})$aryl (the aryl portions of these radicals being optionally substituted by halogen, nitro, hydroxyl, optionally halogenated $(C_1-C_{12})$alkyl or optionally halogenated $(C_1-C_{12})$alkoxy); and the pharmaceutically acceptable salts thereof with acids or bases, it being understood that the compounds below are excluded from the context of the invention:

(a) X = S; n = 0; $R_2$ represents methyl and $R_3$ represents a hydrogen atom; $R_4$ and $R_5$ together form a group $-CR_6=CR_7-$ in which $CR_6$ is linked to X, $R_6$ and $R_7$ together form a $-(CH_2)_3-$ or $-(CH_2)_4-$ chain or alternatively $R_6$ represents a hydrogen atom or a propyl group and $R_7$ is a phenyl group optionally substituted by $-OCH_3$ or a hydroxyl group.

2. Compound according to Claim 1, **characterised in that** X represents -NT in which T is as defined in Claim 1 and $R_4$ and $R_5$ together form $-CR_6=CR_7$.

3. Compound according to Claim 1 or Claim 2, **characterised in that** $R_3$ represents a hydrogen atom.

4. Compound according to any one of Claims 1 to 3, **characterised in that** $R_2$ represents a hydrogen atom or a $(C_6-C_{10})$aryl group optionally substituted by halogen, $(C_1-C_6)$alkoxy, optionally halogenated $(C_1-C_6)$alkyl, nitro and hydroxyl.

5. Compound according to any one of Claims 1 to 4, **characterised in that** n is 0 or 1 and $R_1$ represents a halogen atom.

6. Compound according to any one of Claims 1 and 3 to 5, **characterised in that** X represents S;
$R_4$ and $R_5$ together form a group $-CR_6=CR_7-$ in which
$R_6$ represents a hydrogen atom, $(C_1-C_6)$alkyl, $(C_6-C_{10})$aryl (optionally substituted by halogen, hydroxyl, nitro, $(C_1-C_6)$ alkyl or $(C_1-C_6)$alkoxy), carboxy$(C_1-C_6)$alkyl, or $(C_1-C_6)$alkoxycarbonyl$(C_1-C_6)$alkyl; and
$R_7$ represents a hydrogen atom; hydroxyl; di$(C_1-C_6)$alkylamino-$(C_1-C_6)$alkyl; $(C_1-C_{10})$alkyl; $(C_1-C_6)$alkoxycarbonyl; $(C_6-C_{10})$aryl; heteroaryl; $(C_6-C_{10})$aryi$(C_1-C_6)$alkyl; the aryl and heteroaryl portions of these radicals being optionally substituted by $(C_1-C_6)$alkoxycarbonyl, halogen, hydroxyl, $(C_1-C_6)$alkyl, $(C_6-C_{10})$aryl, (this radical being optionally substituted by halogen, optionally halogenated $(C_1-C_6)$alkyl, $(C_1-C_6)$alkoxy or nitro) or $(C_6-C_{10})$-aryl fused to a 5- to 7-membered aromatic or unsaturated heterocycle containing one, two or three endocyclic hetero atoms chosen from O, N and S; or alternatively $R_6$ and $R_7$ together form an alkylene chain interrupted by a nitrogen atom optionally substituted by $(C_6-C_{10})$aryl$(C_1-C_6)$-alkyl in which the aryl portion is optionally substituted by halogen, optionally halogenated $(C_1-C_6)$alkyl, $(C_1-C_6)$alkoxy, hydroxyl or nitro.

7. Compound according to any one of Claims 1 to 5, **characterised in that** X represents -NT; and $R_4$ and $R_5$ together form a group $-CR_6=CR_7-$ in which $R_6$ represents a hydrogen atom and $R_7$ represents hydroxyl or $(C_6-C_{10})$aryl optionally substituted by halogen, nitro, hydroxyl, optionally halogenated $(C_1-C_6)$alkyl or $(C_1-C_6)$alkoxy.

8. Compound according to Claim 1, chosen from:

3-(biphenyl-4-yl)-5,6-dihydrothiazolo[2,3-b]-1,3-benzodiazepine;
3-(2-furyl)-5,6-dihydrothiazolo[2,3-b]-1,3-benzodiazepine;
3-[4-(ethoxycarbonyl)phenyl]-5,6-dihydrothiazolo[2,3-b]-1,3-benzodiazepine;
3-(biphenyl-3-yl)-5,6-dihydrothiazolo[2,3-b]-1,3-benzodiazepine;
3-(3,4-dihydroxyphenyl)-5,6-dihydrothiazolo[2,3-b]-1,3-benzodiazepine; and
3-(biphenyl-4-yl)-7-chloro-5,6-dihydrothiazolo[2,3-b]-1,3-benzodiazepine.

**9.** Process for the preparation of compounds of the formula I according to Claim 1, in which X represents S and $R_4$ and $R_5$ together form a group $-CR_6=CR_7-$, comprising the reaction of a thione of the formula IIa:

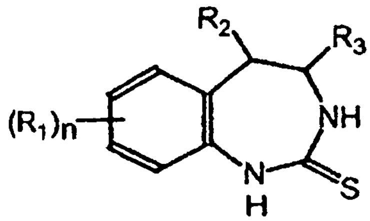

IIa

in which n, $R_1$, $R_2$ and $R_3$ are as defined in Claim 1, with an $\alpha$-haloketone of the formula IVb:

$R_7$-CO-CH$R_6$,-Hal$^3$ IVb

in which $R_6$ and $R_7$ are as defined in Claim 1, and Hal$^3$ represents a halogen atom, in a $C_2$-$C_6$ aliphatic carboxylic acid, at a temperature of between 90 and 130°C.

**10.** Process according to Claim 11, **characterised in that** the aliphatic carboxylic acid is acetic acid.

**11.** Process according to either of Claims 9 and 10, **characterised in that** the temperature is maintained at between 100 and 125°C.

**12.** Process for the preparation of compounds of the formula I according to Claim 1, in which X represents -NH, $R_4$ and $R_5$ together form a group $-CR_6=CR_7-$, and $R_7$ is not hydroxyl, comprising the reaction of a sulfide of the formula V:

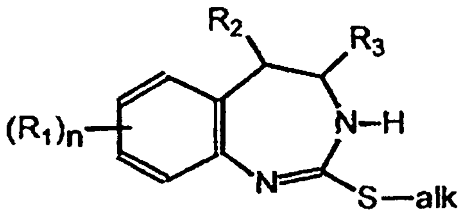

V

in which n, $R_1$, $R_2$ and $R_3$ are as defined in Claim 1, $R_4$ and $R_5$ together form a group $-CR_6=CR_7-$ and alk represents ($C_1$-$C_6$)alkyl, with a protected derivative of the ketone of the formula VI:

$NH_2$-CH$R_6$-CO-$R_7$ VI

in which the carbonyl group is protected with a protecting group that is labile in acidic medium, $R_6$ and $R_7$ being as defined in Claim 1, followed by treatment of the resulting compound with an acid.

**13.** Process for the preparation of compounds of the formula I according to Claim 1, in which X represents -NT in which T is not a hydrogen atom, $R_4$ and $R_5$ together form a group $-CR_6=CR_7$, and $R_7$ represents hydroxyl, comprising the reaction of a sulfide of the formula V:

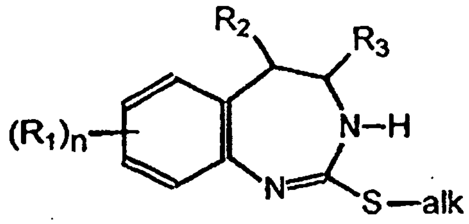

V

in which n, $R_1$, $R_2$ and $R_3$ are as defined in Claim 1, and alk represents $(C_1-C_6)$alkyl, with a derivative of the formula VIII:

$HTN-CHR_6-CO-Y$ VIII

in which T and $R_6$ are as defined in Claim 1 and Y is a leaving group, at a temperature of between 50 and 150°C and preferably at a temperature of between 60 and 100°C.

**14.** Process according to Claim 12, also comprising the reaction of the compound obtained by performing the process of Claim 12, with a halogenated reagent of the formula Hal-T in which T represents $(C_1-C_6)$alkyl, $(C_6-C_{10})$aryl or $(C_6-C_{10})$aryl$(C_1-C_6)$alkyl and Hal is a halogen atom, in the presence of a base, so as to synthesise the corresponding compound of the formula I in which T represents $(C_1-C_6)$alkyl, $(C_6-C_{10})$aryl or $(C_6-C_{10})$-aryl$(C_1-C_6)$alkyl.

**15.** Pharmaceutical composition containing an effective amount of at least one compound of the formula (I) according to any one of Claims 1 to 8, in combination with at least one pharmaceutically acceptable vehicle.

**16.** Use of a compound of the formula I according to any one of Claims 1 to 8, for the preparation of a medicament for the prevention of or treating dyslipidaemia, atherosclerosis and diabetes and its complications.